(19) Europäisches Patentamt / European Patent Office / Office européen des brevets

(11) **EP 3 142 560 B1**

(12) **EUROPEAN PATENT SPECIFICATION**

(45) Date of publication and mention
of the grant of the patent:
**18.08.2021 Bulletin 2021/33**

(51) Int Cl.:
*A61B 8/00* *(2006.01)*    *A61B 8/08* *(2006.01)*
*A61B 8/14* *(2006.01)*

(21) Application number: **15793496.9**

(22) Date of filing: **11.05.2015**

(86) International application number:
**PCT/IB2015/053458**

(87) International publication number:
**WO 2015/173716 (19.11.2015 Gazette 2015/46)**

(54) **MEDICAL-IMAGING SYSTEM AND METHOD THEREOF**

MEDIZINISCHES BILDVERARBEITUNGSSYSTEM UND VERFAHREN DAFÜR

SYSTÈME D'IMAGERIE MÉDICALE ET PROCÉDÉ ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.05.2014 US 201461991899 P**

(43) Date of publication of application:
**22.03.2017 Bulletin 2017/12**

(73) Proprietor: **Exact Imaging Inc.**
**Markham, ON L3R 2N2 (CA)**

(72) Inventors:
• **MAO, Fei**
  **Scarborough, Ontario M1V 1Y3 (CA)**
• **WEN, Jerrold C.**
  **Thornhill, Ontario L3T 5J9 (CA)**
• **WODLINGER, Brian C.**
  **Thornhill, Ontario L4J 8A5 (CA)**
• **AUCOIN, Randy W.**
  **Cavan, Ontario L0A 1C0 (CA)**

• **TORBATIAN, Zahra**
  **Toronto, Ontario M4P 1S2 (CA)**
• **MOONEY, Marina C.**
  **Markham, Ontario L3P 1K1 (CA)**
• **PAVLOVICH, Christian Paul**
  **Baltimore, Maryland 21218 (US)**

(74) Representative: **Viering, Jentschura & Partner mbB**
**Patent- und Rechtsanwälte**
**Am Brauhaus 8**
**01099 Dresden (DE)**

(56) References cited:
| | |
|---|---|
| US-A- 4 819 650 | US-A- 5 353 354 |
| US-A- 6 014 473 | US-A- 6 014 473 |
| US-A- 6 045 508 | US-A1- 2007 038 112 |
| US-A1- 2009 247 879 | US-A1- 2010 280 373 |
| US-A1- 2011 224 551 | US-A1- 2012 238 876 |
| US-A1- 2013 066 207 | US-A1- 2013 102 903 |
| US-A1- 2013 116 548 | |

EP 3 142 560 B1

**Description**

TECHNICAL FIELD

The aspects generally relate to a medical-imaging system and a method thereof.

BACKGROUND

Ultrasonic imaging (sonography) may be used for veterinary medicine and/or human medicine. Diagnostic sonography (ultrasonography) is an ultrasound-based diagnostic imaging technique used for visualizing subcutaneous body structures of a patient, such as tendons, muscles, joints, vessels and internal organs for possible pathology or lesions. Ultrasound images (sonograms) are made by sending a pulse of ultrasound into tissue by using an ultrasound transducer (probe). The sound reflects and echoes off parts of the tissue; the echo (reflected sound) is recorded and displayed as an image to the operator of a medical-imaging system. Generally, the ultrasound transducer is configured to detect objects and measure distances. US 2013/0066207 A1 describes a circuit for driving transducers of an ultrasonic probe of an ultra sound system, wherein timing is based on a rate pulse at a determined rate frequency of 5kHz given by the trigger generation circuit. The ultrasound system performs real time 3D imaging of heart. US 2013/0102903 discloses an ultrasonic diagnostic apparatus including an ultrasonic probe, a position sensor with a position sensor detection unit, an apparatus main body, a display unit and an input unit.

SUMMARY

The invention is defined by the appended claims. All other embodiments are merely exemplary.

Problems associated with known medical-imaging systems were researched. After much study, an understanding of the problem and its solution has been identified.

In order to mitigate, at least in part, the problem(s) associated with known medical-imaging systems, in accordance with an aspect, there is provided a method of operating a medical-imaging system having an ultrasound-transducer interface; the ultrasound-transducer interface is configured to operatively interface with an ultrasound transducer; the ultrasound transducer includes transducer elements; the medical-imaging system also has a spatial sensor configured to provide spatial information indicating spatial movement of the ultrasound transducer; the method includes receiving ultrasound information associated with a scan-line set having a limited number of selectable scan lines of the ultrasound transducer.

In order to mitigate, at least in part, the problem(s) associated with known medical-imaging systems, in accordance with an aspect, there is provided a medical-imaging system including: (A) an ultrasound transducer including transducer elements; (B) an ultrasound-transducer interface configured to operatively interface with the ultrasound transducer; (C) a spatial sensor configured to provide spatial information indicating spatial movement of the ultrasound transducer; and (D) a server configured to receive ultrasound information associated with a scan-line set having a limited number of selectable scan lines of the ultrasound transducer.

In order to mitigate, at least in part, the problem(s) associated with known medical-imaging systems, in accordance with an aspect, there is provided a non-transitory computer-readable medium, including executable code tangibly stored in the non-transitory computer-readable medium; the executable code includes a combination of operational tasks that are executable by a server of a medical-imaging system; the medical-imaging system includes an ultrasound transducer including transducer elements; the medical-imaging system also includes an ultrasound-transducer interface configured to operatively interface with the ultrasound transducer; the medical-imaging system also includes a spatial sensor configured to provide spatial information indicating spatial movement of the ultrasound transducer; the tangibly stored executable code is configured to direct the server to receive ultrasound information associated with a scan-line set having a limited number of selectable scan lines of the ultrasound transducer.

In order to mitigate, at least in part, the problem(s) associated with known medical-imaging systems, in accordance with an aspect, there is provided other aspects as identified in the claims.

Other aspects and features of the non-limiting embodiments may now become apparent to those skilled in the art upon review of the following detailed description of the non-limiting embodiments with the accompanying drawings.

BRIEF DESCRIPTION OF THE DRAWINGS

The non-limiting embodiments may be more fully appreciated by reference to the following detailed description of the non-limiting embodiments when taken in conjunction with the accompanying drawings, in which:

FIG. 1 (SHEET 1/16) depicts a schematic representation of an example of a medical-imaging system;

FIG. 2 (SHEET 2/16) depicts a perspective view of an example of the medical-imaging system of FIG. 1;

FIG. 3A (SHEET 3/16) depicts a schematic representation of an example of an ultrasound transducer of the medical-imaging system of FIG. 1;

FIG. 3B (SHEET 4/16) depicts a schematic representation of an example of the ultrasound transducer of the medical-imaging system of FIG. 1;

FIG. 3C (SHEET 5/16) depicts a schematic representation of an example of an ultrasound transducer of the medical-imaging system of FIG. 1;

FIG. 4 (SHEET 6/16) depicts a schematic representation of a display assembly of the medical-imaging system of FIG. 1;

FIG. 5A (SHEET 7/16) depicts a schematic representation of a flow chart having operations to be included in the executable code to be executed by a server of the medical-imaging system of FIG. 1;

FIG. 5B (SHEET 8/16) depicts an example of an Array [M] stored in a non-transitory computer-readable medium (hereafter referred to as a memory ) of the medical-imaging system of FIG. 1;

FIG. 5C (SHEET 8/16) depicts an example of an Array [Z] stored in a memory of the medical-imaging system of FIG. 1;

FIG. 5D (SHEET 9/16) depicts an example of an Array [Y] stored in a memory of the medical-imaging system of FIG. 1;

FIG. 5E (SHEET 9/16) depicts an example of an Array [X] stored in a memory of the medical-imaging system of FIG. 1;

FIG. 5F (SHEET 10/16) depicts an example of an Array [APEX], an Array [MID] and an Array [BASE] stored in a memory of the medical-imaging system of FIG. 1;

FIG. 5G (SHEET 11/16) depicts an example of an Array [APEX-R] stored in a memory of the medical-imaging system of FIG. 1;

FIG. 5H (SHEET 11/16) depicts an example of an Array [B-mode] stored in a memory of the medical-imaging system of FIG. 1;

FIG. 6 (SHEET 12/16) depicts a display assembly of the medical-imaging system of FIG. 1;

FIG. 7 (SHEET 13/16) depicts a perspective view of an example of an ultrasound transducer of the medical-imaging system of FIG. 1;

FIG. 8 (SHEET 14/16) depicts a schematic example of an operation for collecting a scan line from an ultrasound transducer of the medical-imaging system of FIG. 1;

FIG. 9 (SHEET 15/16) depicts a schematic example of an operation for correcting a change in a spatial position of a scan line obtained from an ultrasound transducer of the medical-imaging system of FIG. 1; and

FIG. 10 (SHEET 16/16) depicts a schematic example of an operation for transforming a pixel along a scan line obtained from an ultrasound transducer of the medical-imaging system of FIG. 1.

The drawings are not necessarily to scale and may be illustrated by phantom lines, diagrammatic representations and fragmentary views. In certain instances, details not necessary for an understanding of the embodiments (and/or details that render other details difficult to perceive) may have been omitted.

Corresponding reference characters indicate corresponding components throughout the several figures of the Drawings. Elements in the several figures are illustrated for simplicity and clarity and have not necessarily been drawn to scale. For example, the dimensions of some of the elements in the figures may be emphasized relative to other elements for facilitating an understanding of the various presently disclosed embodiments. In addition, common, but well-understood, elements that are useful or necessary in commercially feasible embodiments are often not depicted in order to facilitate a less obstructed view of the various embodiments of the present disclosure.

LISTING OF REFERENCE NUMERALS USED IN THE DRAWINGS

| | |
|---|---|
| 100 | medical-imaging system |
| 102 | ultrasound transducer |
| 103 | signal cord |
| 104 | transducer elements |
| 106 | ultrasound-transducer interface |
| 108 | spatial sensor |
| 109 | spatial information |
| 110 | server |
| 112 | memory, non-transitory computer-readable medium |
| 114 | executable code, program, tangibly stored executable code |
| 116 | display assembly |
| 118 | input/output interface module |
| 120 | processor assembly |
| 122 | database |
| 123 | ultrasound data |
| 124 | spatial data |
| 126 | longitudinal axis |
| 128 | reference axis |
| 130 | rotation direction |
| 132 | sound propagation direction |
| 134 | scan line or selectable scan lines or selected scan lines |
| 135 | scan-line set |
| 136 | distal transducer section |
| 138 | medial transducer section |
| 140 | proximal transducer section |
| 142 | basal transverse image |
| 144 | mid transverse image |
| 146 | apex transverse image |
| 148 | B-mode image |
| 200 | flow chart |
| 201 | to 220 operation |
| 222 | transverse image |
| 224 | ray-line |
| 226 | initial position |
| 228 | medial position |
| 230 | final position |
| 232 | transverse plane |
| 234 | current spatial information |
| 236 | relatively small displacement |
| 238 | ray-line |

DETAILED DESCRIPTION OF THE EMBODIMENTS

The following detailed description is merely exemplary in nature and is not intended to limit the described embodiments or the application and uses of the described embodiments. As used herein, the word "exemplary" or "illustrative" means "serving as an example, instance, or illustration." Any implementation described herein as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims. For purposes of the description herein, the terms "upper," "lower," "left," "rear," "right," "front," "vertical," "horizontal," and derivatives thereof shall relate to the examples as oriented in the drawings. Furthermore, there is no intention to be bound by any expressed or implied theory presented in the preceding technical field, background, brief summary or the following detailed description. It is also to be understood that the specific devices and processes illustrated in the attached drawings, and described in the following specification, are simply exemplary embodiments (examples), aspects and/or concepts defined in the appended claims. Hence, specific dimensions and other physical characteristics relating to the embodiments disclosed herein are not to be considered as limiting, unless the claims expressly state

4

otherwise. It is understood that "at least one" is equivalent to "a". The aspects (examples, alterations, modifications, options, variations, embodiments and any equivalent thereof) are described with reference to the drawings. It should be understood that the invention is limited to the subject matter provided by the claims.

According to various embodiments, a method of operating a medical-imaging system comprises an ultrasound-transducer interface being configured to operatively interface with an ultrasound transducer comprising transducer elements, the medical-imaging system further comprising a spatial sensor being configured to provide spatial information indicating spatial movement of the ultrasound transducer and wherein the ultrasound transducer is passed over a surface of a patient, the method comprising: receiving ultrasound information being associated with a scan-line set having a limited number of selectable scan lines of the ultrasound transducer, wherein the limited number is lower than a total number of available scan lines, and wherein the ultrasound-transducer interface is configured to generate the selectable scan lines based on the ultrasound information that was received from the ultrasound transducer and is further configured to allow for sets of selectable scan lines of any grouping or spacing; and increasing a frame rate of the ultrasound transducer by limiting the number of scan lines to the limited number. The method may further comprise receiving spatial information being associated with the scan-line set; and matching the ultrasound information that was received with the spatial information that was received. The method may further comprise identifying a transverse plane extending through the ultrasound transducer. The method may further comprise identifying the selectable scan lines from the scan-line set that correspond to the transverse plane; and displaying the selectable scan lines of the scan-line set that were identified as corresponding to the transverse plane. Identifying the transverse plane may include identifying any one of: a distal transducer section for imaging a base section of a prostate; a medial transducer section for imaging a mid section of the prostate; and a proximal transducer section for imaging an apex section of the prostate. Displaying the selectable scan lines includes may include displaying the selectable scan lines that were identified as corresponding to a distal transducer section, a medial transducer section and a proximal transducer section. Displaying the selectable scan lines may include displaying the selectable scan lines associated with a B-mode image. The method may further comprise correcting aberrant movement of the ultrasound transducer.

According to various embodiments, a medical-imaging system, comprises an ultrasound transducer comprising transducer elements; an ultrasound-transducer interface being configured to operatively interface with the ultrasound transducer, being further configured to generate selectable scan lines based on ultrasound information that was received from the ultrasound transducer and being configured to allow for sets of selectable scan lines of any grouping or spacing; a spatial sensor being configured to provide spatial information indicating spatial movement of the ultrasound transducer; a server being configured to: receive the ultrasound information being associated with a scan-line set having a limited number of the selectable scan lines of the ultrasound transducer, wherein the limited number is lower than a total number of available scan lines; and wherein the system is configured to increase at least in part a frame rate of the ultrasound transducer by limiting the number of scan lines to the limited number. The server may be further configured to: receive the spatial information being associated with the scan-line set; and match the ultrasound information that was received with the spatial information that was received. The server may be further configured to: identify a transverse plane extending through the ultrasound transducer. The server may be further configured to: identify the selectable scan lines of the scan-line set that correspond to the transverse plane; and display the selectable scan lines from the scan-line set that were identified as corresponding to the transverse plane. The server may be further configured to: identify the transverse plane including any one of: a distal transducer section for imaging a base section of a prostate; a medial transducer section for imaging a mid section of the prostate; and a proximal transducer section for imaging an apex section of the prostate. The server may be further configured to: display the selectable scan lines including: displaying the selectable scan lines that were identified as corresponding to a distal transducer section, a medial transducer section and a proximal transducer section. The server may be further configured to: display the selectable scan lines including: displaying the selectable scan lines associated with a B-mode image. The server may be further configured to: correct aberrant movement of the ultrasound transducer.

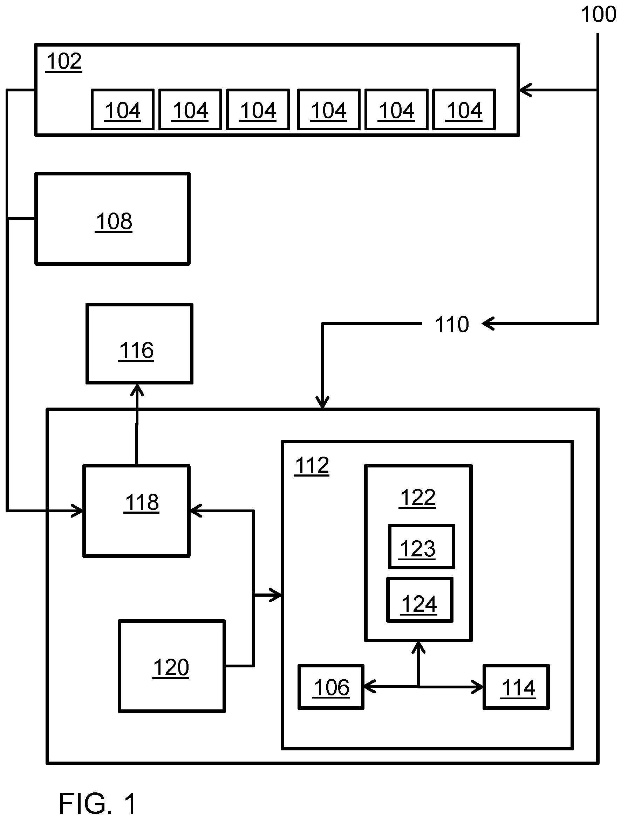

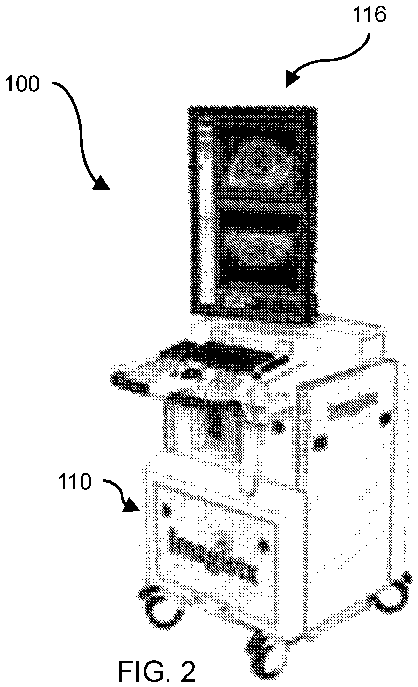

FIG. 1 and FIG. 2 depict a schematic representation and a perspective view, respectively, of examples of a medical-imaging system 100.

The medical-imaging system 100 includes an ultrasound transducer 102 having transducer elements 104, an ultrasound-transducer interface 106, a spatial sensor 108, and a server 110.

The ultrasound transducer 102 is configured to: (A) convert the echo sound signal that was received (by the ultrasound transducer 102) into ultrasound information; and (B) transmit the ultrasound information (via an output port). The ultrasound transducer 102 is also called an ultrasound probe. The ultrasound transducer 102 has the transducer elements 104 arranged in an array; for example, the transducer elements 104 may be aligned along a row, relative to each other, one after the other. The transducer elements 104 are configured to be activated (they may be selectively activated or not activated). The transducer elements 104 are also called transmit and receive elements, in that they transmit ultrasound pulses and receive reflections of the ultrasound pulses. A collection of the transducer elements 104 is also called the transducer array. The ultrasound transducer 102 is also known as an ultrasonic transceiver for the case where the ultrasound transducer 102 is configured to both send (an outgoing ultrasonic pulse) and receive (a reflected ultrasonic pulse). The medical-imaging system 100 uses the ultrasound transducer 102 on a principle similar to radar or sonar, in which the medical-imaging system 100 is configured to evaluate attributes of a target by interpreting the echoes (reflections) from sound waves. The ultrasound transducer 102 is configured to: (A) generate relatively higher frequency sound waves; and (B) receive the echo from the target. The medical-imaging system 100 is configured to: (A) evaluate the ultrasound information provided by the ultrasound transducer 102; (B) calculate the time interval between sending the outgoing signal (from the ultrasound transducer 102) and receiving the echo; (C) determine the distance to the target or an object based on the time interval that was calculated. The ultrasound transducer 102 is configured to generate sound waves in the ultrasonic range, above about generally 18 KHz (Kilo Hertz), by turning electrical energy into sound; then, upon receiving the echo, the ultrasound transducer 102 is configured to turn the reflected sound waves into electrical energy, which can be measured and displayed by the medical-imaging system 100. Ultrasound is an oscillating sound pressure wave with a frequency greater than the upper limit of the human hearing range. Although this limit varies from person to person, it is approximately 20 KHz in healthy, young adults. Some ultrasound devices operate with frequencies from about 20 kHz up to several gigahertz (GHz).

The ultrasound transducer 102 is configured to transmit a signal that includes short bursts of ultrasonic energy. After each burst, the ultrasound transducer 102 is configured to receive a return (reflected) signal within a small window of time corresponding to the time taken for the energy to pass through the tissue of the patient; the signals received during this period then qualify for additional signal processing by the medical-imaging system 100. The ultrasound transducer 102 (medical ultrasonic transducer or probe) may be configured to have any variety of different shapes and sizes for use in making pictures of different parts of the body. According to the inventive methods, the ultrasound transducer 102 is passed over the surface of the body (patient). In other embodiments of the methods disclosed herein not covered by the method of the invention, other ultrasound transducers may be inserted laproscopically, or into an orifice (body opening) of the patient, such as the rectum or vagina. The ultrasound transducer 102 may be configured (by clinicians or operators who perform ultrasound-guided procedures) for use with a probe-positioning system (not depicted and known) configured to hold and/or move the ultrasound transducer 102; the ultrasound transducer 102 includes an array of the transducer elements 104.

The row of the transducer elements 104 of the ultrasound transducer 102 may be aligned in a rectilinear arrangement, or in a curvilinear arrangement. Each of the transducer elements 104 are configured to: (A) transmit an incident sound signal toward a target; and (B) receive an echo sound signal representing sound being reflected back from the target to the transducer elements 104.

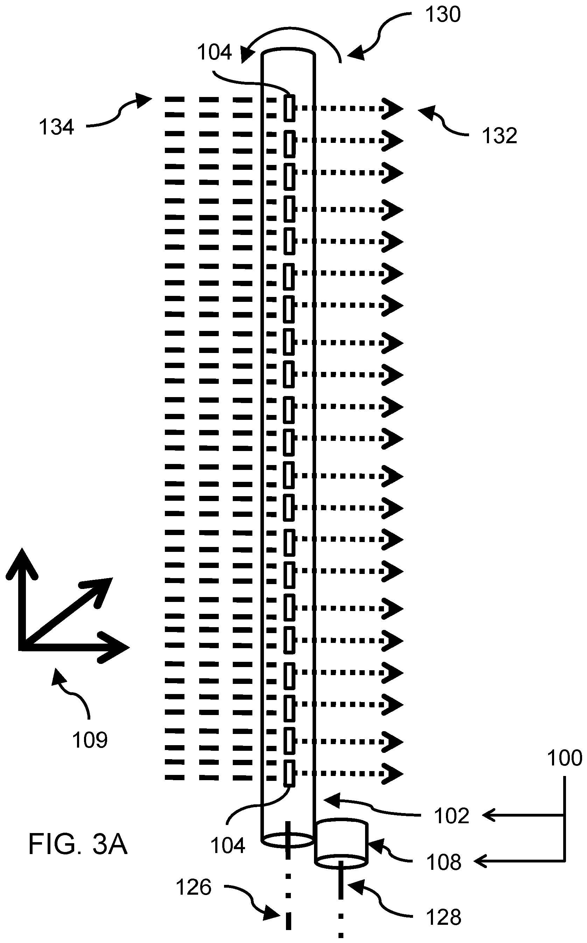

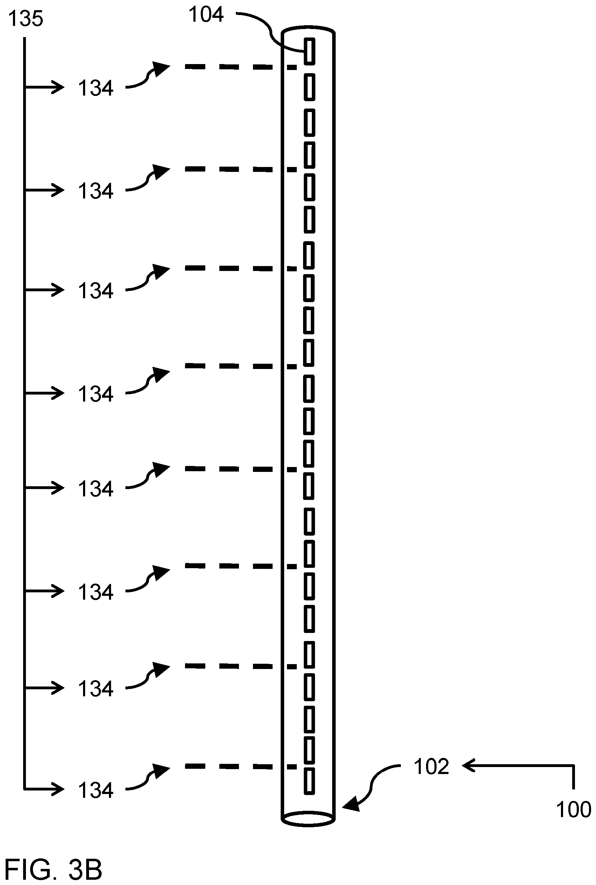

The ultrasound-transducer interface 106 is configured to control operation of the ultrasound transducer 102. The ultrasound-transducer interface 106 is depicted in FIG. 1 as a software program (in accordance with an option). The processor assembly 120 controls the ultrasound transducer 102 via the ultrasound-transducer interface 106. The ultrasound-transducer interface 106 is also called a beam-former. In accordance with an example, the ultrasound-transducer interface 106 may include server-executable code (a software program) tangibly stored in a non-transitory computer-readable medium 112 (hereafter referred to as the memory 112) of the server 110; in accordance with another example, the ultrasound-transducer interface 106 includes a combination of electronic hardware components that cooperate with server-executable code. In general terms, the ultrasound-transducer interface 106 is configured to: (A) operatively connect to the ultrasound transducer 102 (via the output port of the ultrasound transducer 102); (B) control the shape of the incident sound signal to be transmitted by the transducer elements 104; (C) receive the ultrasound information from the ultrasound transducer 102; and (D) provide the scan lines 134 (depicted in FIG. 3A, FIG. 3B) that are mapped to the transducer elements 104 that are activated in such a way as to generate the scan lines 134 to be provided (not all of the transducer elements 104 will be activated and thus these unused instances of the transducer elements 104 will be inactivated). The ultrasound-transducer interface 106 is a device configured to facilitate electronic controlled focusing of the ultrasound energy emitted and/or received by the ultrasound transducer 102.

Generally, the spatial sensor 108 is configured to: (A) detect spatial movement of the ultrasound transducer 102; and (B) provide spatial information 109 (depicted in FIG. 3A) indicating spatial movement of the ultrasound transducer 102 while the ultrasound transducer 102 transmits ultrasound information to the ultrasound-transducer interface 106. The spatial sensor 108 may be attached to the ultrasound transducer 102. Alternatively, the spatial sensor 108 may be integrated with the ultrasound transducer 102.

The server 110 is also known as a computer, etc. Generally, the server 110 is configured to: (A) interface with the ultrasound-transducer interface 106; (B) interface with the spatial sensor 108; and (C) have a memory 112 tangibly storing the executable code 114 (also called processor-executable code, and hereafter referred to as the program 114). The program 114 is a combination of operational tasks to be executed by the server 110. The server 110 is a system that is a combination of software and suitable computer hardware. The server 110 may include a dedicated computer or a combination of computers. The server 110 may be configured for client-server architecture (if so desired).

The memory 112 may refer to the physical devices used to store computer executable programs or processor executable programs (sequences of instructions or operations) and/or data (e.g. program state information) on a temporary basis or a permanent basis for use in the server 110 and anything equivalent thereof. Primary memory is used for the information in physical systems which function at high-speed (such as, RAM or Random Access Memory), as a distinction from secondary memory, which are physical devices for program and data storage which are slow to access but offer higher memory capacity. Primary memory stored on secondary memory is called "virtual memory". By way of example, the memory 112 may include volatile memory and/or non-volatile memory. By way of example, the memory 112 may include secondary memory such as tape, magnetic disks and optical discs (CD-ROM or Compact Disc ROM, and DVD-ROM or Digital Video Disc ROM), etc.

The program 114 is constructed using known software tools as known to those skilled in the art; computer programmed instructions are assembled, in a high level computer programming language, and a complier and other tools are used to convert the computer programmed instructions into the executable code. It will be appreciated that the program 114 provides a method or a sequence of operations to be executed by the processor assembly 120.

The memory 112 includes (tangibly stores) the executable code 114 (also called the program 114). The executable code 114 includes a combination of operational tasks to be executed by the processor assembly 120. For instance, the executable code 114 is configured to direct the server 110 to receive ultrasound information associated with a scan-line set 135 (depicted in FIG. 3B) having a limited number of selectable scan lines 134 of the ultrasound transducer 102. By way of example (and not limited thereto), the scan-line set 135 may have a limited number of scan lines 134 that are mapped with a limited set of the transducer elements 104 of the ultrasound transducer 102 (that were used to generate the selected scan lines 134 of the scan-line set 135), if so desired.

It will be appreciated that in view of the above, there is provided, in general terms, a method of operating the medical-imaging system 100 having the ultrasound-transducer interface 106; the ultrasound-transducer interface 106 is configured to operatively interface with the ultrasound transducer 102; the ultrasound transducer 102 includes transducer elements 104; the medical-imaging system 100 also has the spatial sensor 108 configured to provide spatial information indicating spatial movement of the ultrasound transducer 102; the method includes receiving ultrasound information associated with the scan-line set 135 having the limited number of selectable scan lines 134 of the ultrasound transducer 102. In addition, the server 110 is configured (programmed) to receive ultrasound information associated with a scan-line set 135 having a limited number of selectable scan lines 134 of the ultrasound transducer 102. In addition, the non-transitory computer-readable medium 112 includes executable code 114 that is tangibly stored in the non-transitory computer-readable medium 112; the executable code 114 includes a combination of operational tasks that are executable by the server 110); the executable code 114 is configured (programmed) to direct the server 110 to receive ultrasound information associated with the scan-line set 135 having the limited number of selectable scan lines 134 of the ultrasound transducer 102.

The server 110 also includes a display assembly 116; an input/output interface module 118; a processor assembly 120; a database 122 tangibly stored in the memory 112; ultrasound data 123; and spatial data 124. The ultrasound data 123 and the spatial data 124 are stored in the database 122 or are stored in the memory 112. The input/output interface module 118 is configured to operatively connect the processor assembly 120 with the display assembly 116, the ultrasound-transducer interface 106 (and indirectly, the ultrasound transducer 102) and the spatial sensor 108. In this manner, the processor assembly 120 may control operations of the display assembly 116, the ultrasound-transducer interface 106, and the spatial sensor 108, and also control the ultrasound transducer 102 via direct control of the ultrasound-transducer interface 106. The input/output interface module 118 is also configured to interface the processor assembly 120 with user-interface devices (such as a keyboard, a mouse, a touch-screen display assembly, etc.).

The processor assembly 120 (also called a central processing unit or CPU or a central processor unit) is the hardware within the server 110 that carries out the instructions as set out in the program 114 by performing the arithmetical, logical, and input/output operations. The processor assembly 120 may have one or more instances of the CPU. The CPU may include a microprocessor (meaning the CPU is contained on a single silicon chip). Some integrated circuits (ICs) may contain multiple CPUs on a single chip; those ICs are called multi-core processors. An IC containing a CPU may also contain peripheral devices, and other components of a computer system; this is called a system on a chip (SoC). Components of the CPU are the arithmetic logic unit (ALU), which performs arithmetic and logical operations, and the control unit (CU), which extracts instructions from memory and decodes and executes them, calling on the ALU when necessary. The processor assembly 120 may include an array processor or a vector processor that has multiple parallel computing elements, with no one unit considered the "center". In the distributed computing model, problems are solved by a distributed interconnected set of processors.

The images to be displayed by a medical-imaging system 100 may be displayed in real-time and/or after an acquisition or processing delay (via the display assembly 116).

FIG. 3A depicts a schematic representation of an example of the ultrasound transducer 102 of the medical-imaging system 100 of FIG. 1.

A longitudinal axis 126 extends along the length of the ultrasound transducer 102. A reference axis 128 extends from the spatial sensor 108. The longitudinal axis 126 may be coaxially aligned with, and spaced apart from, the reference axis 128, or may be aligned in any direction that may be convenient or desired. While the spatial sensor 108 provides the spatial information of the spatial sensor 108, the spatial information of the spatial sensor 108 may be mapped in order to provide the spatial information of the ultrasound transducer 102.

A rotation direction 130 indicates the direction of rotation for the ultrasound transducer 102; for instance, the ultrasound transducer 102 is to be rotated around the longitudinal axis 126 by the user, after the ultrasound transducer 102 has been inserted into an orifice of a patient (not depicted).

A sound propagation direction 132 indicates the direction of sound (ultrasonic pulses) propagating from each instance of the transducer elements 104 of the ultrasound transducer 102 toward the target. The sound propagation direction 132 extends (radially) away from the transducer elements 104.

A scan line 134 is generated by the ultrasound-transducer interface 106 of FIG. 1. The instances (number) of the scan lines 134 that are to be generated by the ultrasound-transducer interface 106 depends on the mapping between the number of the scan lines 134 and the number of the transducer elements 104; the mapping may be predefined, and may be a function of the ultrasound-transducer interface 106 of FIG. 1 (as known to those skilled in the art). It is possible for a variable number of the transducer elements 104 to form a single scan line. It is possible to use all instances of the transducer elements 104 to create a limited number of selectable scan lines 134 (such as, four instances of the scan lines 134). It will be appreciated that all of the transducer elements 104 may be activated if so desired and is determined by operation of the ultrasound-transducer interface 106 (known to persons of skill in the art and therefore is not described).

The spatial sensor 108 is configured to provide spatial information 109 in response to movement of the spatial sensor 108. The spatial sensor 108 is associated with the ultrasound transducer 102; for example, the spatial sensor 108 is coupled to (connected to) the ultrasound transducer 102.

Known ultrasound transducer probes are available for trans-rectal or trans-vaginal imaging in a variety of styles, most commonly with a curved-linear array of elements along a tip, known as the "end-fire" ultrasound transducer probe or along a side in a "side-fire" ultrasound transducer probe. Since the end-fire ultrasound transducer probes have their elements at the tip they may be rotated to produce a medical image in various planes; however, the side-fire ultrasound transducer probes have their elements running down a side, and image through the longitudinal axis of the ultrasound transducer 102 (through an anatomical plane of the body of the patient, such as a sagittal plane and/or a coronal plane).

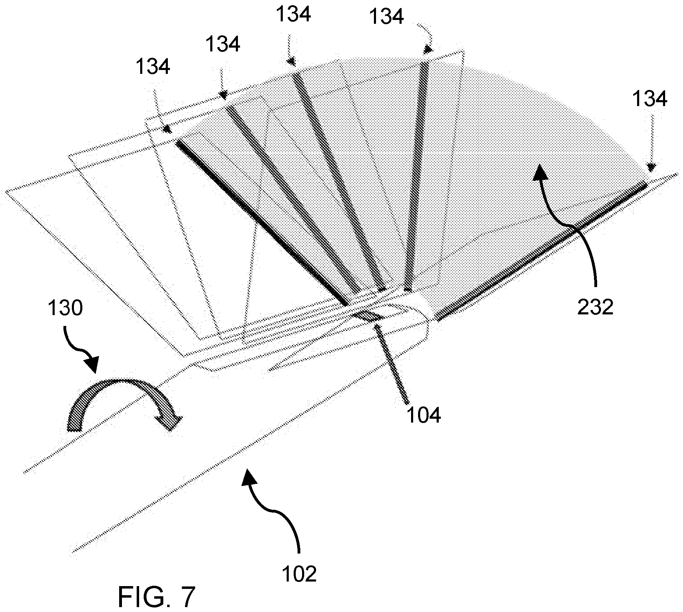

For instance, to obtain a transverse view (reference is made to the example depicted in FIG. 7) from a side-fire ultrasound transducer probe, a three dimensional (3D) reconstruction (a volume) is needed, and a slice of the transverse plane is extracted from the reconstructed 3D volume. The 3D reconstruction-based method requires a sequence of full frame B-Mode ultrasound images (along the sagittal plane), captured by rotating the side-fire ultrasound transducer probe. Acquisition of the 3D information may be slow due to the large number of medical (ultrasound) images required, and also carries a high computation cost (and time) for processing the 3D image reconstruction. Both of these problems become worse as the resolution of the ultrasound images is increased, for example, as in a high-frequency ultrasound imaging system. For example, in order to achieve near real time reconstruction of a transverse image, a relatively higher frame rate (scan rate) is needed and so there must be less data (ultrasound information) to process (in order to accommodate the relatively higher frame rate).

Generally, the medical-imaging system 100 is configured to activate the scan-line set 135 to have a limited number of selectable scan lines 134. The limited number of selectable scan lines 134 are generated by at least one or more or all of the available transducer elements 104 positioned on the ultrasound transducer 102 (such, as the side-fire probe). It is understood that the ultrasound-transducer interface 106 is programmed to (configured to) determine which transducer elements 104 of the ultrasound transducer 102 are to be activated depending on the number of selected (selectable) scan lines 134 and the position of the selected scan lines 134 relative to the longitudinal length of the ultrasound transducer 102. Operation of the ultrasound-transducer interface 106 is known to persons of skill in the art (and therefore is not further described). This arrangement allows for a relatively higher frame rate (scan rate) due to the long "time of flight" of the ultrasound pulse and reflection travelling through the tissue and/or a relatively lower calculation load (calculations to be executed by the processor assembly 120). In this manner, rather than generating a full 3D reconstruction image and selecting a slice of the 3D reconstruction image (in order to obtain the transverse image), the medical-imaging system 100 is configured to generate a smaller fixed (but settable) number of transverse plane images (one, two, three, etc., which represent a limited number of transverse plane images). This is in sharp contrast to the number of transverse plane images that may be obtained from generating the full 3D reconstruction image by using scan lines that are centered at all instances of the transducer elements 104 of the ultrasound transducer 102.

The spatial sensor 108 is configured to assist in the construction of a relatively (reasonably) accurate transverse image. The movement and/or rotation of the ultrasound transducer 102 (or the transducer elements 104) may be freehand (by an operator) or driven by a motor.

The spatial sensor 108 includes, for example, an inertial monitoring unit having a 3-axis accelerometer, a 3-axis gyroscope, and a magnetometer; the spatial sensor 108 is configured to provide 3D position tracking of the ultrasound transducer 102 as the ultrasound transducer 102 is moved (rotated). It will be appreciated that the same performance of the inertial monitoring unit may be provided by a 3-axis accelerometer and/or a 3-axis gyroscope, or using another optical, radio frequency, a mechanical spatial-sensing system or a magnetic spatial-sensing system. A single-axis gyroscope may also be possible, although accuracy may be relatively lower.

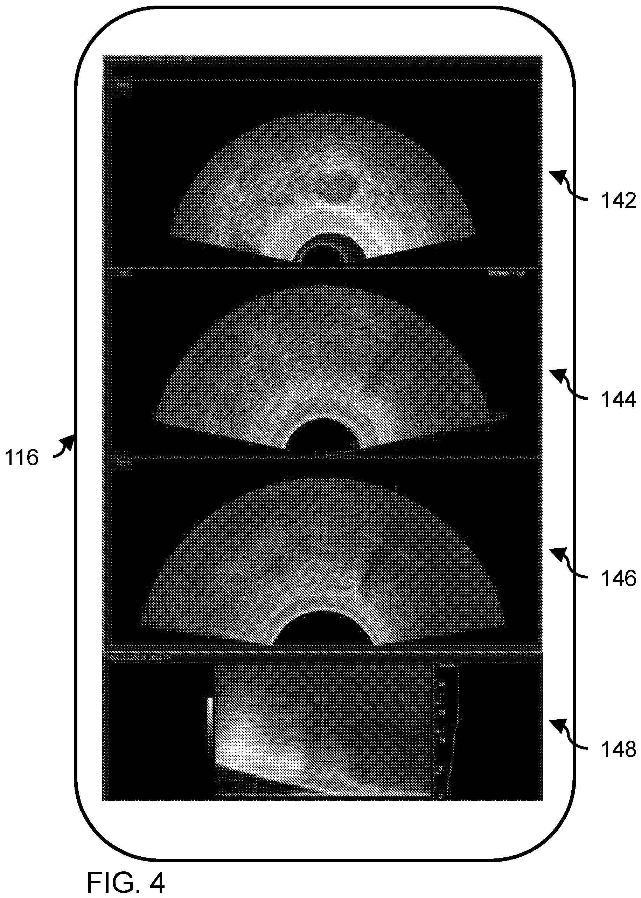

In general terms, [E] separate instances of the transducer elements 104 (located on the ultrasound transducer 102 or the collection of the transducer elements 104) are activated out of a possible total of [F] instances of the transducer elements 104. It will be appreciated that in some cases, all instances of the transducer elements 104 may be activated if so required by the ultrasound-transducer interface 106 depending on the specific instances of the [N] selectable scan lines 134 that are identified by the user or the operator, out of a possible [M] scan lines). The ultrasonic information from the activated instances of the transducer elements 104 are received by the ultrasound-transducer interface 106 via the input/output interface module 118 controlled by the processor assembly 120. The ultrasound-transducer interface 106 is configured to generate the selected scan lines 134 of the scan-line set 135 (to be stored in the memory 112 of FIG. 1) based on the ultrasonic information that was received from the ultrasound transducer 102. The processor assembly 120 is configured to use the scan-line set 135 to construct a limited number of the transverse view (to be displayed to the user or the operator, as depicted in FIG. 4). The result is that a limited number of transverse views (such as three transverse views) are generated or constructed based on the members (the selected scan lines 134) of the scan-line set 135 (at the approximately same time, if so desired). The limited number of transverse views may be generated while the ultrasound transducer 102 is rotated in the patient (either manually by the operator or automatically by a probe-handling machine). In the case of prostate ultrasound imaging in which [N] equals three, the medical-imaging system 100 provides (displays, in real time or near real time, if so desired) the transverse views at the proximal transducer section 140, the medial transducer section 138 and the distal transducer section 136 of the ultrasound transducer 102. For example, a relatively acceptable performance of the medical-imaging system 100 may be achieved with [N] between 1 and 64, and [M] is 1024 For example, there are 512 instances of the transducer element 104 that are used to produce up to 1024 instances of the scan line 134. It is understood that [M] refers to the maximum number of scan lines 134 (where [N] is the number of selected the scan lines 134, and [M] is the total number of available scan lines 134 that may be provided by the ultrasound-transducer interface 106). Once again it is understood that the ultrasound-transducer interface 106 is preprogrammed to determine which of the transducer elements 104 are to be activated in order to provide the ultrasound information for the [N] number of selected scan lines 134. In this manner, the user or the operator is not required to select the specific instances of the transducer elements 104 which are to be activated since the ultrasound-transducer interface 106 performs this function.

In some cases, activating [N] instances of the scan lines 134 may be used to generate K transverse views, where K is less than or equal to N. In these cases, multiple nearby instances of the scan lines 134 are provide to the processor assembly 120, and are used to correct for relatively smaller variations (aberrant or unwanted movement) in the operator's rotational movement of the ultrasound transducer 102; in this manner, image stabilization of the transverse view may be realized. This technique may also correct the transverse view to a planar slice for the case where movement of the ultrasound transducer 102 was not a pure rotational movement (which may otherwise create a curved slice or curved transverse view). The above describes a correction technique or a compensation method; the method is further configured to provide an operation for correcting aberrant (unwanted) movement of the ultrasound transducer 102 (such as the movements caused by the operator).

During acquisition of the transverse image (by the medical-imaging system 100), the ultrasound transducer 102 is rotated (moved) by the user or a motorized system. The acquired instances of the scan lines 134 are selected and displayed (on the display assembly 116, in real time if so desired); however, it may still be useful for the user (operator) to view a B-Mode image on the display assembly 116 so that the user may properly judge the location of the ultrasound transducer 102 within the prostate of the patient. For example, a low-resolution "scout" B-Mode image may be displayed along with the transverse views during transverse acquisition (if so desired) to assist as a guide for the operator handling the ultrasound transducer 102.

An aspect provides the medical-imaging system 100 configured to use a reduced number of instances of the scan lines 134 acquired to [N] (as depicted in FIG. 3B) as a result of activating a limited number of transducer elements 104; [N] is the number that is relatively lower than the total possible number (M) of scan lines 134 (as depicted in FIG. 3A) that may be provided by the ultrasound transducer 102 as a result of activating all of the transducer elements 104. In this manner, the frame rate (scan rate) of the ultrasound transducer 102 is increased (at least in part). In addition, the relatively higher frame rate may allow the medical-imaging system 100 to keep up with the motion of a handheld instance of the ultrasound transducer 102 without any further assistance from stabilization mechanisms (if so desired). This arrangement may also allow a relatively higher resolution in the reconstructed transverse image compared to other freehand techniques that function with a relatively lower frame-rate (and relatively lower image resolution).

The medical-imaging system 100 and/or the operation of the medical-imaging system 100 may be applied to any ultrasound system that uses the ultrasound transducer 102, such as a side-fire ultrasound probe, along with the spatial sensor 108. In most cases, the spatial sensor 108 may be added to the ultrasound transducer 102 (as a retrofit option if so desired).

FIG. 3B depicts a schematic representation of an example of the ultrasound transducer 102 of the medical-imaging system 100 of FIG. 1.

FIG. 3B depicts the ultrasound transducer 102 as a side-fire ultrasound probe with an inertial monitoring unit (an example of the spatial sensor 108) attached to the ultrasound transducer 102.

The program 114 and the ultrasound-transducer interface 106 (of FIG. 1) are configured to cooperate to obtain or receive a limited number of the scan lines 134 (such as 32 selected instances of the scan lines 134 out of a possible 1024 instances of the scan lines 134). By way of example, for the purpose of focusing, up to 128 instances of the transducer elements 104 per instance of a scan line 134 may be used; it will be appreciated that the management of which specific instances of the transducer elements 104 are to be activated is determination is made by the ultrasound-transducer interface 106 in response to the identification of the selected instances of the scan lines 134 of the scan-line set 135. This implies that some instances of the transducer elements 104 are not activated (not used) while other instances of the transducer elements 104 are activated (used). The number of the transducer elements 104 to be activated by the ultrasound-transducer interface 106 will depend on the number of scan lines 134 required by the program 114, and the mapping relationship between the scan lines 134 and the transducer elements 104 of the ultrasound transducer 102. The mapping relationship may be 1:1 (one to one) or any other suitable ratio between the scan lines 134 and the transducer elements 104 (if so desired).

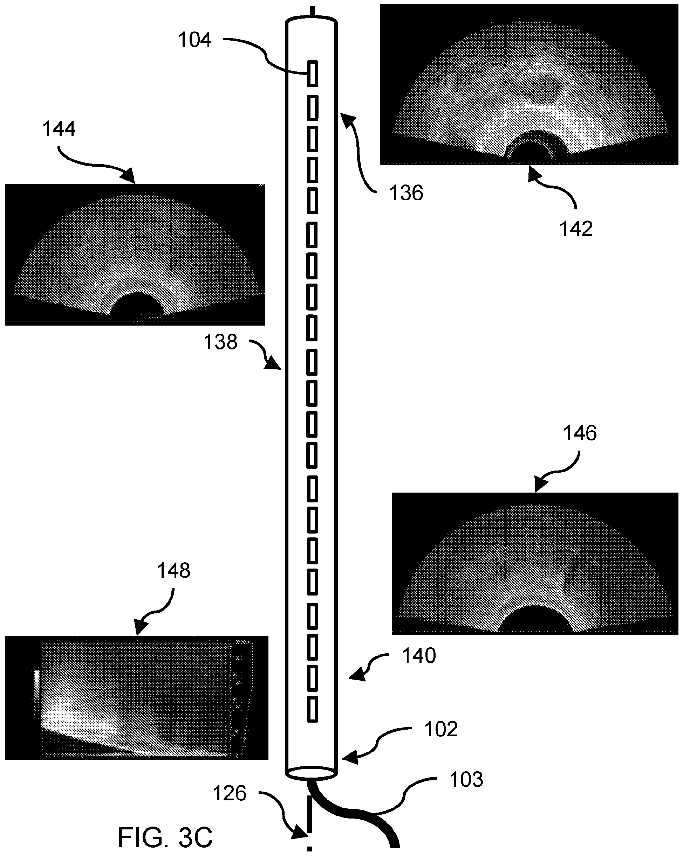

FIG. 3C depicts a schematic representation of an example of the ultrasound transducer 102 of the medical-imaging system 100 of FIG. 1.

The ultrasound transducer 102 includes a distal transducer section 136, a medial transducer section 138 and a proximal transducer section 140.

Generally, the medical-imaging system 100 is configured to provide a transverse view that extends orthogonally from the longitudinal axis 126 of the ultrasound transducer 102 from a selected section of the ultrasound transducer 102. The transverse view is to be displayed on the display assembly 116 of FIG. 1. For example, the medical-imaging system 100 is configured to provide a basal transverse image 142 (base transverse image) that extends orthogonally from the longitudinal axis 126 of the ultrasound transducer 102 from the distal transducer section 136 of the ultrasound transducer 102. The basal transverse image 142 is to be displayed on the display assembly 116 of FIG. 1. For example, the medical-imaging system 100 is configured to provide a mid transverse image 144 that extends orthogonally from the longitudinal axis 126 of the ultrasound transducer 102 from the medial transducer section 138 of the ultrasound transducer 102. The mid transverse image 144 is to be displayed on the display assembly 116 of FIG. 1. For example, the medical-imaging system 100 is configured to provide an apex transverse image 146 (apical transverse image) that extends orthogonally from the longitudinal axis 126 of the ultrasound transducer 102 from the proximal transducer section 140 of the ultrasound transducer 102. The apex transverse image 146 is to be displayed on the display assembly 116 of FIG. 1.

In accordance with an option, the medical-imaging system 100 is configured to provide a B-mode image 148 that extends along the longitudinal axis 126 (the sagittal plane) of the ultrasound transducer 102 from the proximal transducer section 140 of the ultrasound transducer 102 to the distal transducer section 136 of the ultrasound transducer 102. The B-mode image 148 is to be displayed on the display assembly 116 of FIG. 1. The B-mode image 148 displays a two-dimensional cross-section of the tissue (of the patient) being imaged.

FIG. 4 depicts a schematic representation of the display assembly 116 of the medical-imaging system 100 of FIG. 1.

The medical-imaging system 100 is configured to display or provide (via the display assembly 116 of FIG. 1): (A) the basal transverse image 142; (B) the mid transverse image 144; (C) the apex transverse image 146; and (D) the B-mode image 148. In accordance with an option, the medical-imaging system 100 is configured to display (provide) three reconstructed planes (the apex transverse image 146, the mid transverse image 144, and the basal transverse image 142), plus a B-Mode image 148 (also called a scout image, which is located at the bottom section of the display assembly 116).

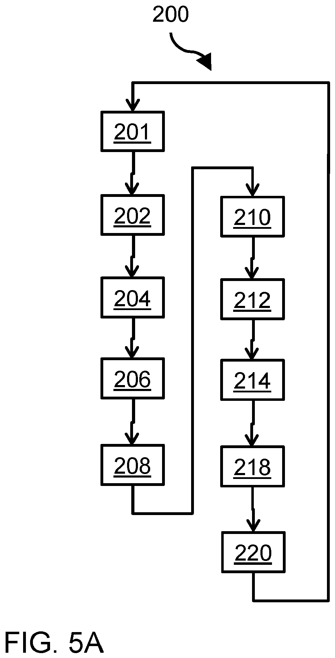

FIG. 5A depicts a schematic representation of a flow chart 200 having operations to be included in the program 114 to be executed by a server 110 of the medical-imaging system 100 of FIG. 1.

Operation 201 includes constructing a spatial orientation map between a transducer orientation of the ultrasound transducer 102 and a sensor orientation of the spatial sensor 108 (operation 201 is executed once). Operation 201 is also called an initialization operation. The construction of the spatial orientation map is known to persons of skill in the art and therefore is not further described here. This initialization also involves identifying the location and orientation of the coordinate system to be used, for example, by setting the initial orientation of the sensor as zero degrees.

According to an option, operation 201 further includes applying a geometric conversion to transform orientation into roll, pitch, and yaw and positional offset about a desired origin. According to an option, operation 201 further includes applying a geometric conversion by using another orientation convention, such as axis-angle and quaternion conventions.

Operational control is passed to operation 202 of FIG. 5A.

Operation 202 includes transmitting a control command (request) to the ultrasound-transducer interface 106.

The control command (request) is configured to instruct the ultrasound-transducer interface 106 to control the ultrasound transducer 102 in such a way that the ultrasound transducer 102 is responsive to the control command. The control command (request) to be transmitted to the ultrasound-transducer interface 106 (from the processor assembly 120 of the server 110) includes an identification of a number of selected scan lines 134 that are members of the scan-line set 135; for example, eight instances (or 32 instances) of the scan lines 134 are identified in the scan-line set 135, and each scan line 134 of the scan-line set 135 are spaced apart from each other, as depicted in FIG. 3B.

The ultrasound-transducer interface 106 is configured to receive the identification of the each scan line 134 of the scan-line set 135, and determines which instances of the transducer elements 104 of the ultrasound transducer 102 are to be activated in order to obtain the ultrasound information for each scan line 134 of the scan-line set 135 from the activated instances of the transducer elements 104. In this manner, the user or the operator need not be concerned with determining which transducer elements 104 are to be activated. The mapping between (A) each scan line 134 of the scan-line set 135 and (B) the activated instances of the transducer elements 104 is determined by the ultrasound-transducer interface 106 of FIG. 1 (in the manner known to those skilled in the art).

The ultrasound-transducer interface 106 is configured to receive the number of selectable (selected) scan lines 134 (of the scan-line set 135) for which ultrasound information is required; the ultrasound-transducer interface 106 then controls specific instances of the transducer elements 104 to be activated in order to obtain the ultrasound information associated with the scan-line set 135. The scan-line set 135 may include instances of the scan lines 134, evenly spaced apart along the longitudinal axis 126 of the ultrasound transducer 102. For example, there may be 512 instances of the transducer elements 104 of the ultrasound transducer 102, and there may be a total number of scan lines (such as, 1024 instances). The instances of the transducer elements 104 (FIG. 3B) to be activated are determined by the ultrasound-transducer interface 106 and the mapping relationship between the scan lines 134 and the transducer elements 104 as known to those skilled in the art.

The ultrasound-transducer interface 106 is configured to allow for selectable scan lines of any selectable grouping or spacing. The scan-line set 135 may include instances of the scan lines 134 that are not evenly spaced apart. For example, scan line_1, scan line_13, and scan line_29. The scan-line set 135 may include instances of the scan lines 134 that are grouped together with selectable group sizes. For example, scan line_1, scan line_2, scan line_3, and scan line_4. The scan-line set 135 may include instances of the scan lines 134 that are not evenly spaced apart and with different group sizes. For example: scan line_1, scan line_2; scan line_14, scan line_15, scan line_16; and, scan line_27, scan line_28, scan line_29, scan line_30, scan line_31.

The scan lines 134 of the scan-line set 135 (as depicted in FIG. 3B) is a subset of the total number (such as, 1024 instances) of the scan lines 134 (as depicted in FIG. 3A).

The ultrasound-transducer interface 106 is configured to activate (pulse) the transducer elements 104 that are required for activation (the remaining instances of the transducer elements 104 are not activated or not pulsed as may be required). The activated instances of the transducer elements 104 are configured to: (A) receive the echo sound signal from the tissue of the patient, and (B) convert the echo sound signal that was received into the ultrasound information, and (C) provided the ultrasound information that was received to the ultrasound-transducer interface 106. In some cases, all of the transducer elements 104 may be activated and receive reflected pulses; The ultrasound-transducer interface 106 is configured to: (A) receive ultrasound information from the activated instances of the transducer elements 104; (B) generate or provide (construct or generate) the scan-line set 135 based on the ultrasound information that was received, and (C) transmit or provide the scan-line set 135 (either to the memory 112 or to the processor assembly 120). In response, the processor assembly 120 writes the scan-line set 135 to the memory 112 (in the ultrasound data 123).

The scan-line set 135 is a subset of a total number of scan lines 134 (such as, 1024). In this manner, the time taken for the medical-imaging system 100 to process the ultrasound data 123 is relatively less than the time taken to manage (process) the ultrasound information that may be (potentially) provided by the total number of the possible instances of the scan lines 134.

The ultrasound transducer 102 is configured to transmit the ultrasound information (for example, enough to build 32 instances of the scan lines 134) to the ultrasound-transducer interface 106. The ultrasound-transducer interface 106 is configured to provide the scan-line set 135 (having, for example, 32 instances of the scan lines 134 as identified or requested by the program 114).

In addition, the control command (request) to be transmitted to the ultrasound-transducer interface 106 includes acoustic focus information. The acoustic focus information is configured to: (A) acoustically focus the incident sound signal to be transmitted from activated instances of the transducer elements 104, and (B) acoustically focus the sound echo signal to be received by the activated instances of the transducer elements 104 from the target (patient tissue). Details regarding acoustic focusing for the ultrasound transducer 102 are known to persons of skill in the art and therefore are not further described here.

The acoustic focus information includes (for example) a focus number (F#) configured to indicate a degree of focus (e.g., the degree of tight focus; the ratio of aperture to depth, etc.) to be used to focus the activated instances of the transducer elements 104 that are mapped to the scan-line set 135 (as depicted in FIG. 3B). The acoustic focus information includes (for example): (A) a transmit focal depth for the incident sound signal to be transmitted toward the target by the activated instances of the transducer elements 104; and/or (B) a receive focal depth, or a series of receive focal depths, for the echo sound signal representing sound reflected back from the target to the activated instances of the transducer elements 104. Wherein, with a dynamic receive focal depth, a series of receive focal depths are received by the activated instances of the transducer elements 104.

The activated instances of the transducer elements 104 are configured to: (A) transmit the incident sound signal (a sound pulse); (B) receive the sound echo signal (the signal is reflected back from the tissue of the patient); and (C) convert the sound echo signal that was received into the ultrasound information. The ultrasound transducer 102 is configured to transmit the ultrasound information to the ultrasound-transducer interface 106 of FIG. 1.

Operational control is passed to operation 204 of FIG. 5A.

Operation 204 is executed while the ultrasound transducer 102 is rotated from a starting position (such as, the plus 70 degree rotation position) to an ending position (such as, the minus 70 degree rotation position) by the operator (user) of the ultrasound transducer 102. Operation 204 includes receiving (reading) groupings of the ultrasound information, in accordance with the scan rate of the ultrasound transducer 102, from the ultrasound-transducer interface 106 in response to the ultrasound-transducer interface 106 receiving the ultrasound information from the ultrasound transducer 102. For example, each grouping includes 32 instances (selected instances) of the scan lines 134 for the scan-line set 135.

The scan-line set 135 may have, for example, 32 selected instances of the scan lines 134, which are selected from the total number of possible scan lines 134 (such as, 1024 total possible instances of the scan lines 134.)

The ultrasound-transducer interface 106 is configured to manage the required activation of instances of the transducer elements 104 in such a way that the ultrasound-transducer interface 106 provides ultrasound information for the scan-line set 135 (and not the ultrasound information for all possible instances of the scan lines 134 of the scan-line set 135); therefore, the effective scan rate of the ultrasound transducer 102 is much higher relative to obtaining the ultrasonic information from all instances of the scan lines 134.

Operation 204 further includes tagging (associating) the ultrasound information (each scan-line set 135) that was received with a time stamp (the time that the scan-line set 135 was received), for each scan-line set 135 that was collected while the ultrasound transducer 102 is rotated from plus 70 degrees to minus 70 degrees. For instance, the scan-line set 135 includes 32 instances of the scan lines 134 per scan-line set 135 (or any other desired number of scan lines 134).

Operation 204 further includes storing the ultrasound information (such as, the 32 instances of the selected scan lines 134 in a scan-line set 135) for each scan along the longitudinal axis 126 of the ultrasound transducer 102 (as the ultrasound transducer 102 is rotated) along with the time stamp in the memory 112.

The processor assembly 120 is configured to store the ultrasound information to the memory 112 in the form of an Array [M].

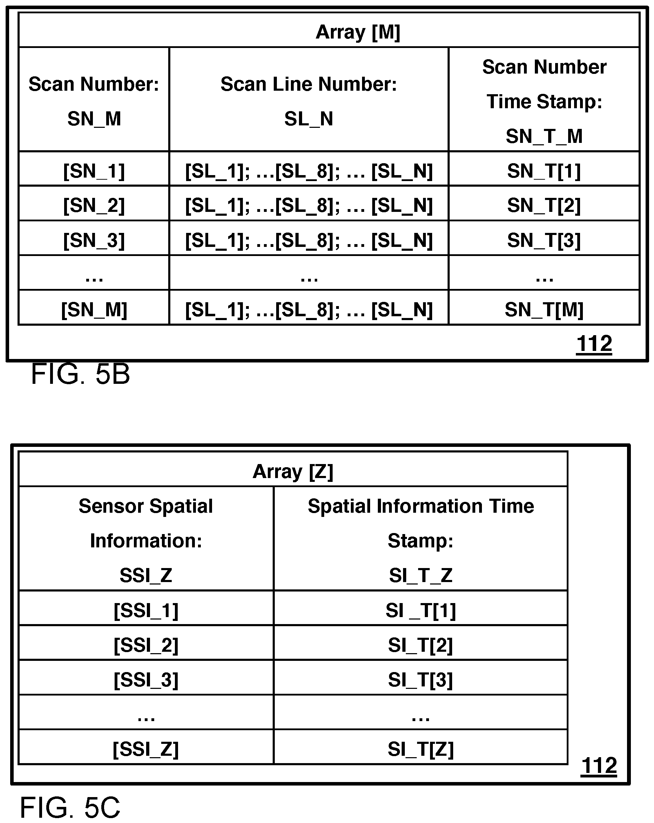

FIG. 5B depicts an example of the Array [M] stored in the memory 112 of the medical-imaging system 100 of FIG. 1.

The Array [M] includes data organized (for example) into sections, such as: [Scan Number: SN_M] section; [Scan Line Number: SL_N] section; and [Scan Number Time Stamp: SN_T_M] section. The [Scan Number: SN_M] section contains the scan number (one scan number per scan-line set 135 having [N] instances of the scan lines 134. [N] equals the number of selected scan lines 134 in the scan-line set 135 of FIG. 3B, while FIG. 3A depicts all instances of the scan lines 134 that may be provided by the ultrasound-transducer interface 106.

The [Scan Line Number: SL_N] section contains the number of scan lines 134 in the scan-line set 135, such as, eight instances of the scan lines 134 per scan-line set 135 as depicted in FIG. 3B.

The [Scan Number Time Stamp: SN_T_M] section contains the time stamp for time when the scan number [M] was completed for a scan-line set 135 and provided by the ultrasound-transducer interface 106 to the server 110.

For instance, scan number [SN_1] section starts at plus 70 degrees (scan start); this represents a start time for ultrasound information collection for movement (rotation) of the ultrasound transducer 102. Scan number [SN_M] section ends at minus 70 degrees (scan end); this represents an end time for ultrasound information collection for movement (rotation) of the ultrasound transducer 102.

For instance, for the case where the ultrasound transducer 102 is manually held by the operator of the medical-imaging system 100, the ultrasound transducer 102 is moved (rotated) in response to movement of the operator's hand; movement of the operator's hand while manually manipulating movement (rotation) of the ultrasound transducer 102 while ultrasound information is collected from start time to end time may result in unpredictable positioning of the ultrasound transducer 102 over time. For example, the operator may dwell at a spatial position of the ultrasound transducer 102 during movement thereof, and thus may result in an over collection of ultrasound information. Nevertheless, the ultrasound information is collected from the ultrasound-transducer interface 106 and is stored to the memory 112 of FIG. 1.

Operational control is passed to operation 206 of FIG. 5A.

Operation 206 includes transmitting a sensor-command request to the spatial sensor 108. The sensor-command request is configured to instruct the spatial sensor 108 to transmit the sensor-spatial information back to the server 110 (while the ultrasound transducer 102 is spatially moved).

By way of example, an API interface (a known software technique) may be configured to operatively interface the spatial sensor 108 to the server 110. In accordance with a first option, the sensor-command request is configured to request the spatial sensor 108 to transmit the sensor-spatial information on a continuous basis to the server 110, and the server 110 receives the sensor-spatial information from the spatial sensor 108 on a continuous basis (threaded computing). In accordance with a second option, each time the ultrasound information is received from the ultrasound transducer 102, the sensor-command request is configured to request the spatial sensor 108 to transmit the sensor-spatial information, on an as-needed basis, to the server 110.

Operational control is passed to operation 208 of FIG. 5A.

Operation 208 includes receiving (reading) the sensor-spatial information from the spatial sensor 108. Operation 208 further includes tagging (associating) the sensor-spatial information that was received from the spatial sensor 108 with a time stamp (time when received). Operation 208 further includes storing (writing), to the memory 112, the sensor-spatial information that was received from the spatial sensor 108 along with the time stamp tagged to the sensor-spatial information.

The processor assembly 120 is configured to receive the spatial information from the spatial sensor 108 via the input/output interface module 118; the processor assembly 120 is configured to store the spatial information to the memory 112 in the form of an Array [Z]. The Array [Z] includes: [Sensor Spatial Information: SSI_Z]; and [Spatial Information Time Stamp: SI_T_Z].

FIG. 5C depicts an example of the Array [Z] stored in the memory 112 of the medical-imaging system 100 of FIG. 1.

The array [Z] includes data organized (for example) into sections, such as: [Sensor Spatial Information: SSI Z] section; and [Spatial Information Time Stamp: SI_T_Z] section.

For instance, before the processor assembly 120 begins collecting (receiving) the ultrasound information from the ultrasound transducer 102 via the ultrasound-transducer interface 106, the processor assembly 120 receives (reads) the sensor-spatial information from the spatial sensor 108, via the input/output interface module 118, and then writes (provides) the sensor-spatial information to the memory 112 (into the spatial data 124 or the [Sensor Spatial Information: SSI Z] section), along with a time stamp (into the [Spatial Information Time Stamp: SI_T_Z] section, which indicates the time that spatial information was received by the processor assembly 120 from the spatial sensor 108.

The spatial information contained in the Array [Z] includes the sensor-spatial information collected during the rotation of the ultrasound transducer 102 from plus 70 degrees to minus 70 degrees.

Operational control is passed to operation 210 of FIG. 5A.

Operation 210 includes computing (determining) the transducer-spatial information of the ultrasound transducer 102 using: (A) the sensor-spatial information obtained (received) from the spatial sensor 108, and (B) the spatial orientation map that was constructed in operation 201. The result of the computation may be written (stored) to an Array [Y] to the memory 112 of FIG. 1.

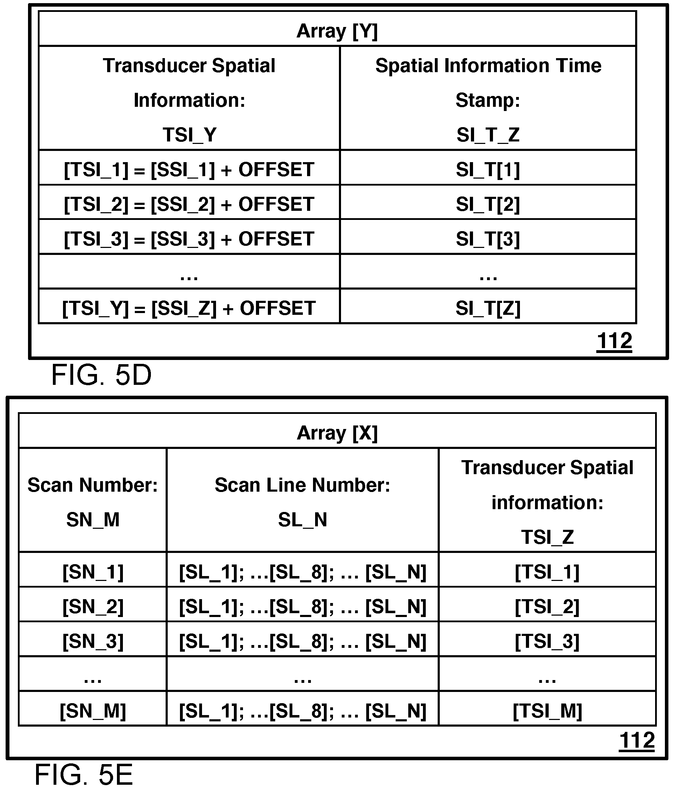

FIG. 5D depicts an example of the Array [Y] stored in the memory 112 of the medical-imaging system 100 of FIG. 1.

The Array [Y] includes data organized (for example) into sections, such as: [Transducer Spatial Information: TSI_Y] section, and [Spatial Information Time Stamp: SI_T_Z] section.

For instance, the processor assembly 120 is configured to compute the transducer-spatial information by using FORMULA {1}:

FORMULA {1}: [Transducer Spatial Information: TSI_Y] section = [Sensor Spatial Information: SPI_Z] section PLUS [an offset provide by a spatial orientation map] section.

Operation 210 further includes storing (writing), to the memory 112, the transducer-spatial information that was computed for the ultrasound transducer 102, along with the time stamp tagged with the sensor-spatial information of the spatial sensor 108. The processor assembly 120 computed the spatial information for the ultrasound transducer 102 by using FORMULA {1}. The time stamp for each [Transducer Spatial Information: TSI Z] section is the same time stamp for the [Sensor Spatial Information: SSI_Z] section used in the Array [Z].

The spatial information contained in the Array [Y] includes the transducer-spatial information that was computed from the sensor-spatial information collected during the rotation of the ultrasound transducer 102 from plus 70 degrees (start of scan) to minus 70 degrees (end of scan).

Operational control is passed to operation 212 of FIG. 5A.

Prior to executing the operation 212, the following is the data stored to (and available from) the memory 112 of FIG. 1: the Array [Z] and the Array [Y]. The Array [Z] and the Array [Y] each have time stamps.

Operation 212 includes matching up the ultrasound information provided in the Array [Z] with the transducer-spatial information provided in the Array [Y] by matching up the time stamps associated with the Array [Z] and the Array [Y]. In this manner, the transducer-spatial information is associated with the corresponding scan-line number, which is an instance of the scan line 134 depicted in FIG. 3B.

Operation 212 includes writing (storing) the matched information, in the form of the Array [X] to the memory 112 of FIG. 1.

FIG. 5E depicts an example of the Array [X] stored in the memory 112 of the medical-imaging system 100 of FIG. 1.

The Array [X] includes data organized (for example) into sections, such as: [Scan Number: SN_M] section, [Scan Line Number: SL_N] section, and [Transducer Spatial information: TSI Z] section.

Generally, the operation 212 includes receiving spatial information associated with the scan-line set 135.

In accordance with an option (if so desired), operation 212 includes executing an interpolation algorithm in order to achieve good synchronicity (best fit) when matching up the information to be inserted in the Array [X]. Interpolation algorithms are well known to persons of skill in the art, and therefore are not described in detail.

Operational control is passed to operation 214 of FIG. 5A.

Operation 214 includes correcting for a change in position and orientation (spatial information) of ultrasound transducer 102 as the ultrasound transducer 102 was rotated from: (A) a baseline (the baseline is the spatial information at plus 70 degrees or the start position at the start of the movement or rotation of the ultrasound transducer 102) to (B) an end position of rotation (terminus) by using the spatial transducer information.

Operation 214 further includes identifying a transverse plane (at least one or more transverse planes) that is aligned transverse (perpendicular, across) to the axis of the ultrasound transducer 102. For example, FIG. 3C identifies three examples of the transverse plane that may extend through the distal transducer section 136, the medial transducer section 138 and the proximal transducer section 140.

Operation 214 further includes, for each scan-line set 135 (depicted in FIG. 3B) having a limited number (selected or selectable instances) of the scan lines 134 that were collected and stored in the memory 112, determining (identifying) which instances of the scan line 134 of the scan-line set 135 are spatially positioned closest to the transverse plane that was identified.

Operation 214 further includes tagging (marking, selecting) each scan line in the scan-line set 135 that was determined to be spatially positioned closest to the transverse plane (of interest).

For instance, with reference to FIG. 3B and FIG. 3C, a signal cable 103 extends from the ultrasound transducer 102. The distal transducer section 136 is associated with scan line_1, the medial transducer section 138 is associated with scan line_16, and the proximal transducer section 140 is associated with scan line_32. An Array [APEX], an Array [MID] and an Array [BASE] may be constructed if so desired to contain the ultrasound information.

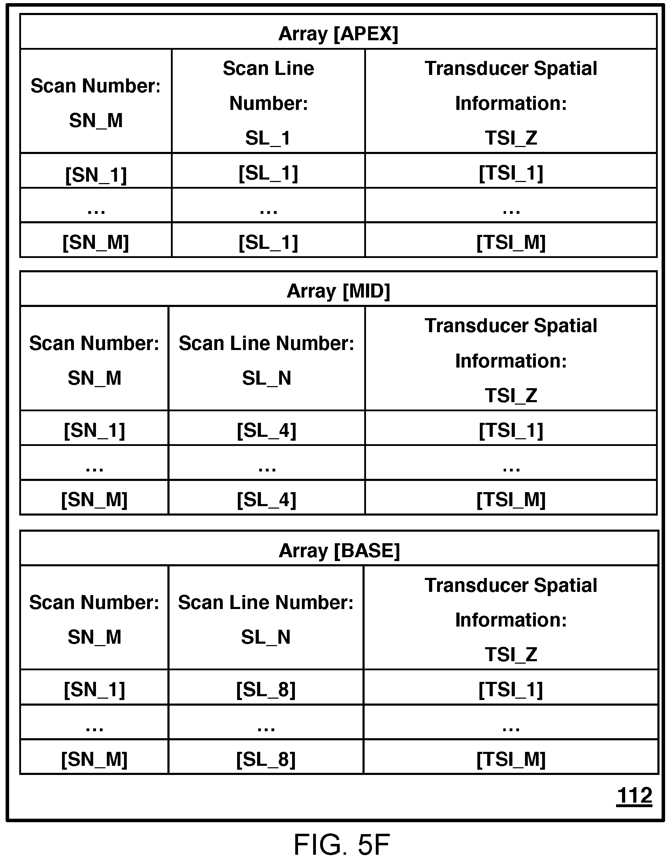

FIG. 5F depicts an example of the Array [APEX], the Array [MID] and the Array [BASE] stored in the memory 112 of the medical-imaging system 100 of FIG. 1.

The Array [APEX] includes data organized (for example) into sections, such as: [Scan Number: SN_M] section; [Scan Line Number: SL_1] section, and [Transducer Spatial Information: TSI_Z] section. The Array [MID] includes data organized (for example) into sections, such as: [Scan Number: SN_M] section; [Scan Line Number: SL_1] section, and [Transducer Spatial Information: TSI Z] section. The Array [BASE] includes data organized (for example) into sections, such as: [Scan Number: SN_M] section; [Scan Line Number: SL_1] section, and [Transducer Spatial Information: TSI Z] section.

Generally, operation 214 includes identifying the selectable scan lines 134 from the scan-line set 135 that correspond to the transverse plane.

It may be expected that the operator may inadvertently move the ultrasound transducer 102 along unwanted directions during the collection of the ultrasound information. For instance, for the case where the operator has inserted the ultrasound transducer 102 into an orifice of the patient, and the operator inadvertently moves the ultrasound transducer 102 along the longitudinal axis 126 of the ultrasound transducer 102 by a scan-line pitch (the distance between adjacent instances of the scan lines 134 depicted in FIG. 3B), and the scan line_1, the scan line_2, and the scan line_3 were available with the transverse plane originally centered on scan line 2, then the operation 214 includes selecting (tagging) the scan line_1 or the scan line_3 (in the scan-line set 135) depending on the direction of the spatial movement of the ultrasound transducer 102. This is considered to be a way to correct for unwanted movements of the ultrasound transducer 102 during collection of the ultrasound information.

Operational control is passed to operation 216 of FIG. 5A.

Operation 216 includes associating (identifying) a number (Q) of ray-lines to be used in the construction of the transverse plane.

For instance, the transverse plane is discretized into even increments (such as, 0.5 degrees or increments from plus 70.0 degrees to minus 70.0 degrees) into which the ray-lines may be positioned.

Operation 216 includes using the transducer-spatial information (such as, the roll angle from spatial information) to match (identify) (A) the nearest scan number [SN_M] to be selected for the scan line number to be used in the construction of the transverse plane (such as, SL_1 associated with the distal transducer section 136 of FIG. 3C) with (B) the nearest ray-line to be displayed on the display assembly 116 of FIG. 1. An Array [APEX-R] may be constructed and stored in the memory 112 of FIG. 1 (if so desired) to contain the matches found in operation 216.

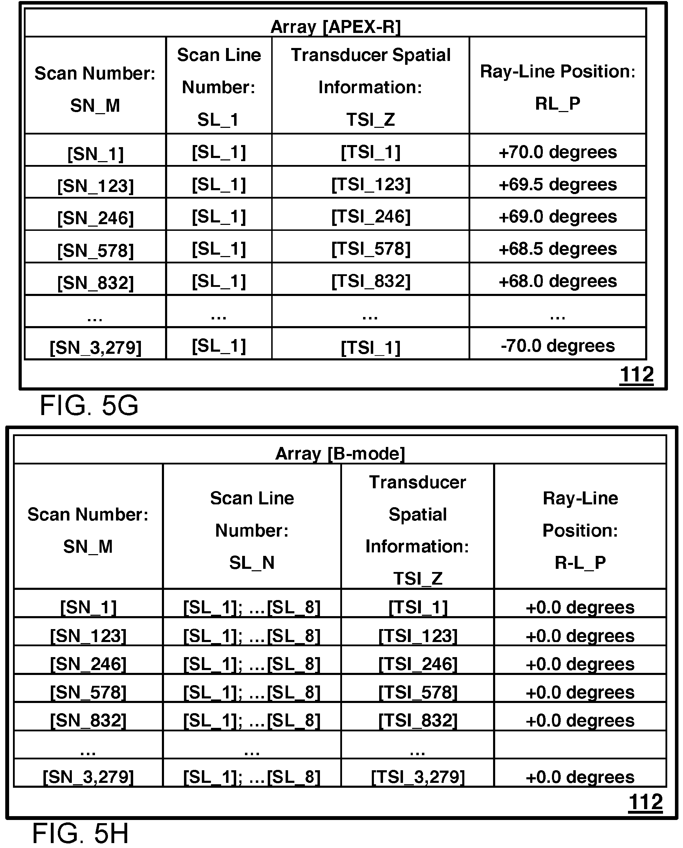

FIG. 5G depicts an example of the Array [APEX-R] stored in the memory 112 of the medical-imaging system 100 of FIG. 1.

The Array [APEX-R] includes data organized (for example) into sections, such as: [Scan Number: SN_M] section; [Scan Line Number: SL_1] section; [Transducer Spatial Information: TSI_Z] section; and [Ray-Line Position: RL_P] section. The Array [APEX-R] is for the apex section of the ultrasound transducer 102. Similar set up may be performed for an Array [MID-R] for the medial section of the ultrasound transducer 102, and an Array [BASE-R] for the base section of the ultrasound transducer 102 (or any other desired section of the ultrasound transducer 102).

For example, for the case where the measured roll was plus 44.3 degrees and the transverse plane was discretized into 0.5 degree increments, the ray-line positioned at plus 44.5 degrees is selected (tagged) to be populated with the data from the scan line 134 for each frame being reconstructed. Alternatively, in another embodiment, a scan-conversion or interpolation approach may be used to map the scan lines onto ray-lines for display.

Operational control is passed to operation 218 of FIG. 5A.

Operation 218 includes mapping (interpolating) the scan-line data, from the Array [APEX-R] for example, onto the ray-line using a relationship identified by the following FORMULA {2}:

$$\text{FORMULA \{2\}:} \qquad \{X\} = (\,[a]+[d]\,)\cos(t), \ \{Y\} = (\,[a]+[d]\,)\sin(t)$$

where:

$\{X\}$ and $\{Y\}$ are the transverse-plane image coordinates;
$[a]$ is the position along the ultrasound line (along the axis of the ultrasound transducer 102);
$[d]$ is the distance from the axis (axis of rotation of the ultrasound transducer 102); and
$(t)$ is the orientation of the ray-line in the transverse plane.

Operational control is passed to operation 220 of FIG. 5A.

Operation 220 includes displaying, to the display device of the server 110, the transverse plane(s) using the mapping provided by operation 216. For example, operation 220 includes displaying any one of the b-mode image 148, the mid transverse image 144, and the basal transverse image 142 (in any combination and permutation thereof) as depicted in FIG. 4.

An option of operation 220 includes constructing and displaying a B-mode image based on the instances of the scan-line set 135 collected during the rotation of the ultrasound transducer 102. The B-mode is an image that spans along the axis of the ultrasound transducer 102 from the base of the ultrasound transducer 102 to the apex of the ultrasound transducer 102. It will be appreciated that the Array [B-mode] can be constructed.

It will be appreciated that the updating (populating) of each instance of the ray-line may be accomplished in various ways; for example, (A) each instance of the ray-line may be populated for each iteration through the flow chart of FIG. 5A, or (B) all instances of the ray-lines may be populated in a single iteration through the flow chart of FIG. 5A.

For example, for the case where a single instance of the ray-line is populated, then operational control is directed back to operation 202. For the case where the ultrasound transducer 102 is rotated between plus 70 degrees to minus 70 degrees, a full image may be constructed; however, for the case where real-time display is required, the display assembly 116 of FIG. 4 may be updated with each ray-line on a line-by-line basis (if so desired).

FIG. 5H depicts an example of the Array [B-mode] stored in the memory 112 of the medical-imaging system 100 of FIG. 1.

Operational control is passed to operation 202 of FIG. 5A.

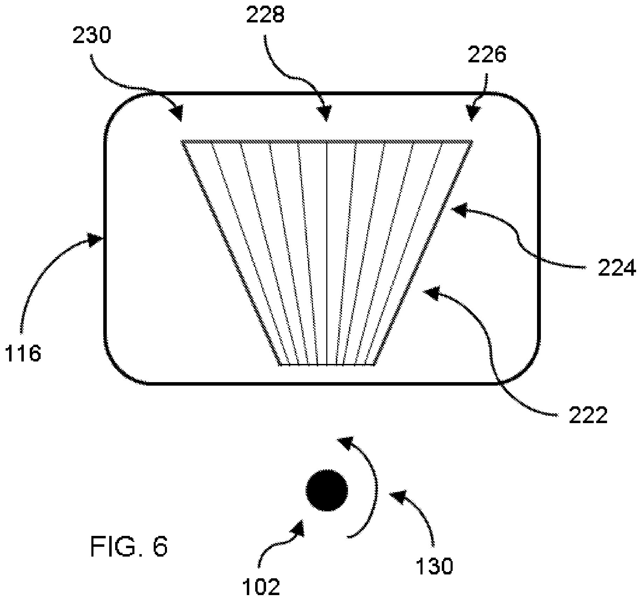

FIG. 6 depicts the display assembly 116 of the medical-imaging system 100 of FIG. 1.

The transverse image 222 is depicted on the display assembly 116. The ultrasound transducer 102 is depicted below the display assembly 116. The transverse image 222 includes instances of a ray-line 224 placed side-by-side. The transverse image 222 spans from an initial position 226 (plus 70 degrees) to a medial position 228 (zero degrees) to final position 230 (minus 70 degrees).

FIG. 7 depicts a perspective view of an example of the ultrasound transducer 102 of the medical-imaging system 100 of FIG. 1.

By way of example, the ultrasound transducer 102 includes a high-resolution side-fire ultrasound probe having a linear array of the transducer elements 104. A single instance of the transducer elements 104 is depicted. The transverse plane 232 is constructed with the ultrasound information provided by the ultrasound transducer 102. A single instance of the scan line 134 is captured with a single instance of the transducer elements 104 of the ultrasound transducer 102, and is used to construct a single instance of the transverse plane 232. The medical-imaging system 100 is configured to construct the transverse plane 232 (having a transverse image) from individual instances of the scan lines 134 that were collected during movement (rotation) of the ultrasound transducer 102.

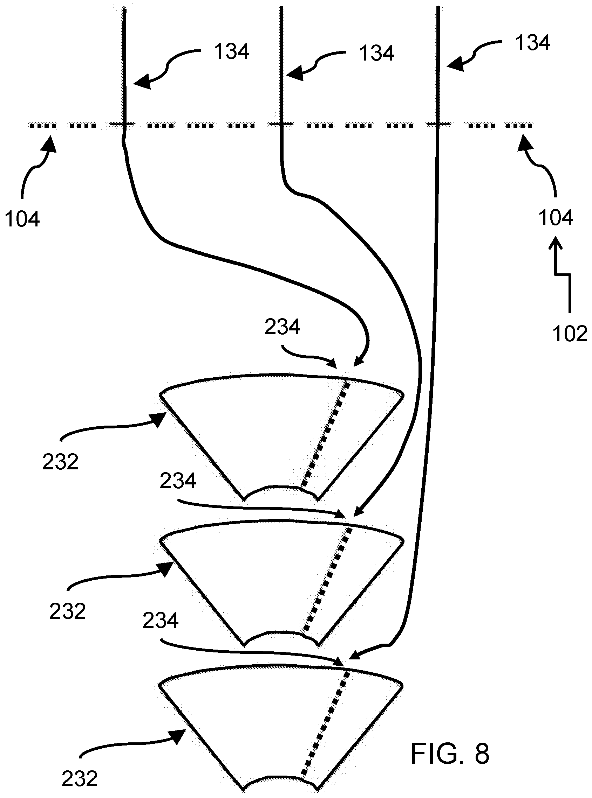

FIG. 8 depicts a schematic example of an operation for collecting a scan line 134 from the ultrasound transducer 102 of the medical-imaging system 100 of FIG. 1.

For example, at a given point in time, the processor assembly 120 queries the spatial sensor 108 for the current spatial information 234 (such as, position and/or angle). At the same time (at approximately the same time), three instances of the scan lines 134 (representing ultrasound information) are received from the array of the transducer elements 104, and recorded (written to memory 112 of FIG. 1). The scan lines 134 are then placed into the appropriate locations in the three instances of the transverse reconstruction images located in the transverse plane 232.

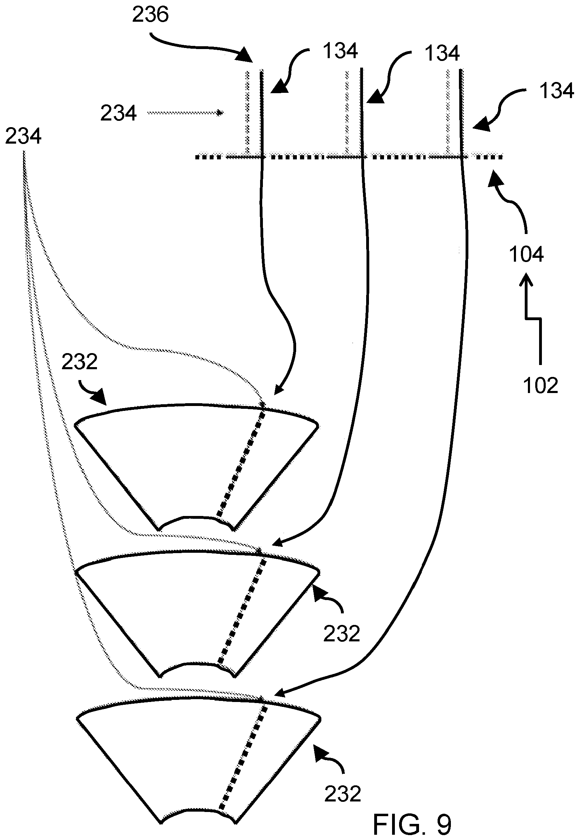

FIG. 9 depicts a schematic example of an operation for correcting position (spatial) change of a scan line 134 obtained from the ultrasound transducer 102 of the medical-imaging system 100 of FIG. 1.

There is a relatively small displacement 236 as a result of inadvertent movement of the ultrasound transducer 102 having the transducer elements 104 (as a result, for example, of operator error). At a given point in time, the processor assembly 120 queries the spatial sensor 108 (of FIG. 1) for the current spatial information 234 (position and angle, spatial information). At the same time (approximately the same time), several instances of the scan lines 134 (ultrasound information) are received from the transducer elements 104; the active instances of the transducer elements 104 are depicted in a relatively blacker line, while the inactive instances of the transducer elements 104 are depicted in dashed lines. The scan lines 134 that best compensate for any positional changes are then placed into the appropriate locations in the three transverse plane 232 (also called a reconstruction image).

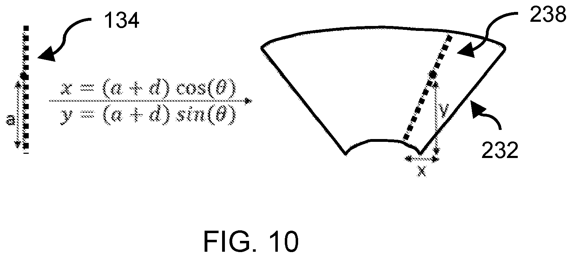

FIG. 10 depicts a schematic example of an operation for transforming a pixel along a scan line 134 obtained from the ultrasound transducer 102 of the medical-imaging system 100 of FIG. 1.

A pixel "a" is obtained from the scan line 134, and then the pixel "a" is displayed on a ray-line 238 positioned in the transverse plane 232 (to be displayed on the display assembly 116 of FIG. 1). The pixel "a" is transformed along a single instance of the scan line 134 to the corresponding pixel "a" positioned on the transverse plane 232.

Any one or more of the technical features and/or any one or more sections of the technical features of the medical-imaging system 100 may be combined with any other one or more of the technical features and/or any other one or more sections of the technical features of the medical-imaging system 100 in any combination and/or permutation. Any one or more of the technical features and/or any one or more sections of the technical features of the medical-imaging system 100 may stand on its own merit without having to be combined with another other technical feature.

This written description uses examples to disclose the invention, to enable any person skilled in the art to make and use the invention. The patentable scope of the invention is defined by the claims.

## Claims

1. A method of operating a medical-imaging system (100) comprising an ultrasound-transducer interface (106) being configured to operatively interface with an ultrasound transducer (102) comprising transducer elements (104), the medical-imaging system further comprising a spatial sensor (108) being configured to provide spatial information (109) indicating spatial movement of the ultrasound transducer (102) and wherein the ultrasound transducer is passed over a surface of a patient, the method comprising:

   receiving ultrasound information being associated with a scan-line set (135) having a limited number of selectable scan lines of the ultrasound transducer (102), wherein the limited number is lower than a total number of available scan lines (134), and wherein the ultrasound-transducer interface (106) is configured to generate the selectable scan lines based on the ultrasound information that was received from the ultrasound transducer and is further configured to allow for sets of selectable scan lines of any grouping or spacing; and
   increasing a frame rate of the ultrasound transducer (102) by limiting the number of scan lines to the limited number.

2. The method of claim 1, further comprising:

   receiving spatial information (109) being associated with the scan-line set (135); and
   matching the ultrasound information that was received with the spatial information (109) that was received.

3. The method of claim 2, further comprising:
   identifying a transverse plane (232) extending through the ultrasound transducer (102).

4. The method of claim 3, further comprising:

identifying the selectable scan lines from the scan-line set (135) that correspond to the transverse plane (232); and

displaying the selectable scan lines of the scan-line set (135) that were identified as corresponding to the transverse plane (232).

5. The method of claim 3 or claim 4, wherein:
identifying the transverse plane (232) includes identifying any one of:

a distal transducer section (136) for imaging a base section of a prostate;
a medial transducer section (138) for imaging a mid section of the prostate; and
a proximal transducer section (140) for imaging an apex section of the prostate.

6. The method of any one of claims 1 to 5, wherein:
displaying the selectable scan lines includes:
displaying the selectable scan lines that were identified as corresponding to a distal transducer section (136), a medial transducer section (138) and a proximal transducer section (140).

7. The method of any one of claims 1 to 6, wherein:
displaying the selectable scan lines includes:
displaying the selectable scan lines associated with a B-mode image.

8. The method of any one of claims 1 to 7, further comprising:
correcting aberrant movement of the ultrasound transducer (102).

9. A medical-imaging system (100), comprising:

an ultrasound transducer (102) comprising
transducer elements (104);
an ultrasound-transducer interface (106) being configured to operatively interface with the ultrasound transducer (102), being further configured to generate selectable scan lines based on ultrasound information that was received from the ultrasound transducer and being configured to allow for sets of selectable scan lines of any grouping or spacing;
a spatial sensor (108) being configured to provide spatial information (109) indicating spatial movement of the ultrasound transducer (102);
a server (110) being configured to:

receive the ultrasound information being associated with a scan-line set (135) having a limited number of the selectable scan lines of the ultrasound transducer (102), wherein the limited number is lower than a total number of available scan lines (134); and
wherein the system (100) is configured to increase at least in part a frame rate of the ultrasound transducer (102) by limiting the number of scan lines to the limited number.

10. The medical-imaging system (100) of claim 9, wherein:
the server (110) is further configured to:

receive the spatial information (109) being associated with the scan-line set (135); and
match the ultrasound information that was received with the spatial information (109) that was received.

11. The medical-imaging system (100) of claim 9 or claim 10, wherein:
the server (110) is further configured to:
identify a transverse plane (232) extending through the ultrasound transducer (102).

12. The medical-imaging system (100) of claim 11, wherein:
the server (110) is further configured to:

identify the selectable scan lines of the scan-line set (135) that correspond to the transverse plane (232); and
display the selectable scan lines from the scan-line set (135) that were identified as corresponding to the transverse plane (232).

**13.** The medical-imaging system (100) of claim 11 or claim 12, wherein:
the server (110) is further configured to:
identify the transverse plane (232) including any one of:

a distal transducer section (136) for imaging a base section of a prostate;
a medial transducer section (138) for imaging a mid section of the prostate; and
a proximal transducer section (140) for imaging an apex section of the prostate.

**14.** The medical-imaging system (100) of any one of claims 9 to 13, wherein:
the server (110) is further configured to:
display the selectable scan lines including:
displaying the selectable scan lines that were identified as corresponding to an distal transducer section, a medial transducer section and a proximal transducer section.

**15.** The medical-imaging system (100) of any one of claims 9 to 14, wherein:
the server (110) is further configured to:
display the selectable scan lines including:
displaying the selectable scan lines associated with a B-mode image.

**16.** The medical-imaging system (100) of any one claims 9 to 15, wherein:
the server (110) is further configured to:
correct aberrant movement of the ultrasound transducer (102).

**Patentansprüche**

**1.** Ein Verfahren zum Betreiben eines medizinischen Bildgebungssystems (100) umfassend eine Ultraschallwandler-Schnittstelle (106), die so konfiguriert ist, dass sie mit einem Ultraschallwandler (102) mit Wandlerelementen (104) operativ gekoppelt ist, das medizinische Bildgebungssystem ferner einen räumlichen Sensor (108) umfassend, der konfiguriert ist, um räumliche Informationen (109) bereitzustellen, die eine räumliche Bewegung des Ultraschallwandlers (102) anzeigen, und wobei der Ultraschallwandler über eine Oberfläche eines Patienten geführt wird, das Verfahren umfassend:

Empfangen von Ultraschallinformationen, die einem Scanline-Satz (135) mit einer begrenzten Anzahl auswählbarer Scanlinien des Ultraschallwandlers (102) zugeordnet sind, wobei die begrenzte Anzahl geringer ist als eine Gesamtzahl verfügbarer Scanlinien (134), und wobei die Ultraschallwandler-Schnittstelle (106) so konfiguriert ist, dass sie die auswählbaren Scanlinien basierend auf den Ultraschallinformationen erzeugt, die von dem Ultraschallwandler empfangen wurden, und ferner konfiguriert ist, um Sätze von auswählbaren Scanlinien beliebiger Gruppierung oder Abstandes zu ermöglichen; und
Erhöhen einer Frame-Frequenz des Ultraschallwandlers (102) durch Begrenzen der Anzahl von Scanlinien auf die begrenzte Anzahl.

**2.** Das Verfahren nach Anspruch 1, ferner umfassend:

Empfangen von räumlichen Informationen (109), die dem Scanline-Satz (135) zugeordnet sind; und
Abgleichen der empfangenen Ultraschallinformationen mit den empfangenen räumlichen Informationen (109).

**3.** Das Verfahren nach Anspruch 2, ferner umfassend:
Identifizieren einer Querebene (232), die sich durch den Ultraschallwandler (102) erstreckt.

**4.** Das Verfahren nach Anspruch 3, ferner umfassend:

Identifizieren der auswählbaren Scanlinien aus dem Scanline-Satz (135), die der Querebene (232) entsprechen; und
Anzeigen der auswählbaren Scanlinien des Scanline-Satzes (135), die als der Querebene (232) entsprechend identifiziert wurden.

**5.** Das Verfahren nach Anspruch 3 oder Anspruch 4, wobei:

Identifizieren der Querebene (232) das Identifizieren eines von:

einem distalen Wandlerabschnitt (136) zum Abbilden eines Basisabschnitts einer Prostata;
einen medialen Wandlerabschnitt (138) zum Abbilden eines mittleren Abschnitts der Prostata; und
einen proximalen Wandlerabschnitt (140) zum Abbilden eines Scheitelabschnitts der Prostata

beinhaltet.

6. Das Verfahren nach einem der Ansprüche 1 bis 5, wobei:
Anzeigen der auswählbaren Scanlinien umfasst:
Anzeigen der auswählbaren Scanlinien, die als einem distalen Wandlerabschnitt (136), einem medialen Wandlerabschnitt (138) und einem proximalen Wandlerabschnitt (140) entsprechend identifiziert wurden.

7. Das Verfahren nach einem der Ansprüche 1 bis 6, wobei:
Anzeigen der auswählbaren Scanlinien umfasst:
Anzeigen der auswählbaren Scanlinien, die einem B-Modus-Bild zugeordnet sind.

8. Das Verfahren nach einem der Ansprüche 1 bis 7, ferner umfassend:
Korrigieren einer abweichenden Bewegung des Ultraschallwandlers (102).

9. Ein medizinisches Bildgebungssystem (100), umfassend:

einen Ultraschallwandler (102) umfassend Wandlerelemente (104);
eine Ultraschallwandler-Schnittstelle (106), die dazu konfiguriert ist, mit dem Ultraschallwandler (102) operativ gekoppelt zu sein, die ferner dazu konfiguriert ist, auswählbare Scanlinien basierend auf Ultraschallinformationen zu erzeugen, die von dem Ultraschallwandler empfangen wurden, und die konfiguriert ist, um Sätze von auswählbaren Scanlinien beliebiger Gruppierung oder Abstände zu ermöglichen;

einen räumlichen Sensor (108), der konfiguriert ist, um räumliche Informationen (109) bereitzustellen, die die räumliche Bewegung des Ultraschallwandlers (102) anzeigen;
ein Server (110), der konfiguriert ist, um:
Ultraschallinformationen zu empfangen, die einem Scanline-Satz (135) zugeordnet sind, der eine begrenzte Anzahl der auswählbaren Scanlinien des Ultraschallwandlers (102) aufweist, wobei die begrenzte Anzahl geringer ist als eine Gesamtzahl verfügbarer Scanlinien (134); und
wobei das System (100) konfiguriert ist, um zumindest teilweise eine Frame-Frequenz des Ultraschallwandlers (102) zu erhöhen, indem die Anzahl von Scanlinien auf die begrenzte Anzahl begrenzt wird.

10. Das medizinische Bildgebungssystem (100) nach Anspruch 9, wobei:
der Server (110) ferner konfiguriert ist, um:

räumliche Informationen (109) zu empfangen, die dem Scanline-Satz (135) zugeordnet sind; und
die empfangenen Ultraschallinformationen mit den empfangenen räumlichen Informationen (109) abzugleichen.

11. Das medizinische Bildgebungssystem (100) nach Anspruch 9 oder Anspruch 10, wobei:
der Server (110) ferner konfiguriert ist, um:
eine Querebene (232) zu identifizieren, die sich durch den Ultraschallwandler (102) erstreckt.

12. Das medizinische Bildgebungssystem (100) nach Anspruch 11, wobei:

der Server (110) ferner konfiguriert ist, um:
die auswählbaren Scanlinien des Scanline-Satzes (135) identifizieren, die der Querebene (232) entsprechen; und
die auswählbaren Scanlinien aus dem Scanline-Satz (135) anzuzeigen, die als der Querebene (232) entsprechend identifiziert wurden.

13. Das medizinische Bildgebungssystem (100) nach Anspruch 11 oder Anspruch 12, wobei:
der Server (110) ferner konfiguriert ist, um:
die Querebene (232), beinhaltend einen der folgenden:

einen distalen Wandlerabschnitt (136) zum Abbilden eines Basisabschnitts einer Prostata;
einen medialen Wandlerabschnitt (138) zum Abbilden eines mittleren Abschnitts der Prostata; und
einen proximalen Wandlerabschnitt (140) zum Abbilden eines Scheitelabschnitts der Prostata

zu identifizieren.

14. Das medizinische Bildgebungssystem (100) nach einem der Ansprüche 9 bis 13, wobei:
der Server (110) ferner konfiguriert ist, um:
die auswählbaren Scanlinien anzuzeigen, beinhaltend:
Anzeigen der auswählbaren Scanlinien, die als einem distalen Wandlerabschnitt, einem medialen Wandlerabschnitt und einem proximalen Wandlerabschnitt entsprechend identifiziert wurden.

15. Das medizinische Bildgebungssystem (100) nach einem der Ansprüche 9 bis 14, wobei:
der Server (110) ferner konfiguriert ist, um:
die auswählbaren Scanlinien anzuzeigen, beinhaltend:
Anzeigen der auswählbaren Scanlinien, die einem B-Modus-Bild zugeordnet sind.

16. Das medizinische Bildgebungssystem (100) nach einem der Ansprüche 9 bis 15, wobei:
der Server (110) ferner konfiguriert ist, um:
eine abweichende Bewegung des Ultraschallwandlers (102) zu korrigieren.

**Revendications**

1. Procédé d'utilisation d'un système d'imagerie médicale (100) comprenant une interface de transducteur à ultrasons (106) configurée pour s'interfacer opérationnellement avec un transducteur à ultrasons (102) comprenant des éléments de transducteur (104), le système d'imagerie médicale comprenant en outre
un capteur spatial (108)
configuré pour fournir des informations spatiales (109) indiquant un mouvement dans l'espace du transducteur à ultrasons (102) et dans lequel le transducteur à ultrasons est passé sur une surface d'un patient,
le procédé comprenant :

la réception d'informations ultrasonores
associées à un ensemble de ligne de balayage (135) présentant un nombre limité de lignes de balayage du transducteur à ultrasons (102) pouvant être sélectionnées, dans lequel le nombre limité
est inférieur à un nombre total de lignes de balayage disponibles (134), et dans lequel l'interface du transducteur à ultrasons (106) est configurée pour générer les lignes de balayage pouvant être sélectionnées sur la base des informations ultrasonores qui ont été reçues du transducteur à ultrasons et est en outre configurée pour permettre des ensembles de lignes de balayage pouvant être sélectionnées de tout groupement ou espacement ; et
l'augmentation d'une fréquence de trame du transducteur à ultrasons (102) en limitant le nombre de lignes de balayage au nombre limité.

2. Procédé de la revendication 1, comprenant en outre :

la réception d'informations spatiales (109) associées à l'ensemble de ligne de balayage (135) ; et
la mise en correspondance des informations ultrasonores qui ont été reçues avec les informations spatiales (109) qui ont été reçues.

3. Procédé de la revendication 2, comprenant en outre :
l'identification d'un plan transversal (232) s'étendant à travers le transducteur à ultrasons (102).

4. Procédé de la revendication 3, comprenant en outre :

l'identification des lignes de balayage pouvant être sélectionnées depuis l'ensemble de ligne de balayage (135) qui correspondent au plan transversal (232) ; et
l'affichage des lignes de balayage pouvant être sélectionnées de l'ensemble de ligne de balayage (135) qui a été identifié comme correspondant au plan transversal (232).

5. Procédé selon la revendication 3 ou la revendication 4, dans lequel :
l'identification du plan transversal (232) comprend l'identification de l'une quelconque de :

une section de transducteur distale (136) pour l'imagerie d'une section de base d'une prostate ;
une section de transducteur médiane (138) pour l'imagerie d'une section centrale d'une prostate ; et
une section de transducteur proximale (140) pour l'imagerie d'une section supérieure d'une prostate.

6. Procédé de l'une quelconque des revendications 1 à 5, dans lequel :
l'affichage des lignes de balayage pouvant être sélectionnées comprend :
l'affichage des lignes de balayage pouvant être sélectionnées qui ont été identifiées comme correspondant à une section de transducteur distale (136), une section de transducteur médiane (138) et une section de transducteur proximale (140).

7. Procédé de l'une quelconque des revendications 1 à 6, dans lequel :
l'affichage des lignes de balayage pouvant être sélectionnées comprend :
l'affichage des lignes de balayage pouvant être sélectionnées associées à une image en mode B.

8. Procédé de l'une quelconque des revendications 1 à 7, comprenant en outre :
la correction d'un mouvement aberrant du transducteur à ultrasons (102).

9. Système d'imagerie médicale (100) comprenant :

un transducteur à ultrasons (102) comprenant des éléments de transducteur (104) ;
une interface de transducteur à ultrasons (106) configurée pour s'interfacer opérationnellement avec le transducteur à ultrasons (102), qui est en outre configurée pour générer des lignes de balayage pouvant être sélectionnées sur la base d'informations ultrasonores qui ont été reçues du transducteur à ultrasons et configurée pour permettre des ensembles de lignes de balayage pouvant être sélectionnées de tout groupement ou espacement ;
un capteur spatial (108) configuré pour fournir des informations spatiales (109) indiquant un mouvement dans l'espace du transducteur à ultrasons (102) ;
un serveur (110) configuré pour :

recevoir les informations ultrasonores associées à un ensemble de ligne de balayage (135) présentant un nombre limité des lignes de balayage du transducteur à ultrasons (102) pouvant être sélectionnées,
dans lequel le nombre limité est inférieur à un nombre total de lignes de balayage disponibles (134) ; et
dans lequel le système (100) est configuré pour augmenter au moins en partie une fréquence de trame du transducteur à ultrasons (102) en limitant le nombre de lignes de balayage au nombre limité.

10. Système d'imagerie médicale (100) de la revendication 9, dans lequel :
le serveur (110) est en outre configuré pour :

recevoir des informations spatiales (109) associées à l'ensemble de ligne de balayage (135) ; et
mettre en correspondance des informations ultrasonores qui ont été reçues avec les informations spatiales (109) qui ont été reçues.

11. Système d'imagerie médicale (100) de la revendication 9 ou de la revendication 10, dans lequel :
le serveur (110) est en outre configuré pour :
Identifier un plan transversal (232) s'étendant à travers le transducteur à ultrasons (102).

12. Système d'imagerie médicale (100) de la revendication 11, dans lequel :
le serveur (110) est en outre configuré pour :

identifier des lignes de balayage pouvant être sélectionnées de l'ensemble de ligne de balayage (135) qui correspondent au plan transversal (232) ; et
afficher les lignes de balayage pouvant être sélectionnées à partir de l'ensemble de ligne de balayage (135) qui a été identifié comme correspondant au plan transversal (232).

**13.** Système d'imagerie médicale (100) de la revendication 11 ou de la revendication 12, dans lequel :

le serveur (110) est en outre configuré pour :

identifier le plan transversal (232) comprenant l'identification de l'une quelconque de :

une section de transducteur distale (136) pour l'imagerie d'une section de base d'une prostate ;
une section de transducteur médiane (138) pour l'imagerie d'une section centrale d'une prostate ; et
une section de transducteur proximale (140) pour l'imagerie d'une section supérieure d'une prostate.

**14.** Système d'imagerie médicale (100) de l'une quelconque des revendication 9 à 13, dans lequel :

le serveur (110) est en outre configuré pour :

afficher les lignes de balayage pouvant être sélectionnées comprenant :

l'affichage des lignes de balayage pouvant être sélectionnées qui ont été identifiées comme correspondant à une section de transducteur distale, une section de transducteur médiane et une section de transducteur proximale.

**15.** Système d'imagerie médicale (100) de l'une quelconque des revendications 9 à 14, dans lequel :

le serveur (110) est en outre configuré pour :

afficher les lignes de balayage pouvant être sélectionnées comprenant :

l'affichage des lignes de balayage pouvant être sélectionnées associées à une image en mode B.

**16.** Système d'imagerie médicale (100) de l'une quelconque des revendications 9 à 15, dans lequel :

le serveur (110) est en outre configuré pour :

corriger un mouvement aberrant du transducteur à ultrasons (102).

100

102

104 104 104 104 104 104

108

116

110

112

118

122

123

120

124

106 114

FIG. 1

FIG. 2

FIG. 3A

FIG. 3B

FIG. 3C

FIG. 4

200

201

202

204

206

208

210

212

214

218

220

FIG. 5A

| Array [M] | | |
|---|---|---|
| Scan Number: SN_M | Scan Line Number: SL_N | Scan Number Time Stamp: SN_T_M |
| [SN_1] | [SL_1]; ...[SL_8]; ... [SL_N] | SN_T[1] |
| [SN_2] | [SL_1]; ...[SL_8]; ... [SL_N] | SN_T[2] |
| [SN_3] | [SL_1]; ...[SL_8]; ... [SL_N] | SN_T[3] |
| ... | ... | ... |
| [SN_M] | [SL_1]; ...[SL_8]; ... [SL_N] | SN_T[M] |

<u>112</u>

FIG. 5B

| Array [Z] | |
|---|---|
| Sensor Spatial Information: SSI_Z | Spatial Information Time Stamp: SI_T_Z |
| [SSI_1] | SI _T[1] |
| [SSI_2] | SI_T[2] |
| [SSI_3] | SI_T[3] |
| ... | ... |
| [SSI_Z] | SI_T[Z] |

<u>112</u>

FIG. 5C

| Array [Y] | |
|---|---|
| Transducer Spatial Information: TSI_Y | Spatial Information Time Stamp: SI_T_Z |
| [TSI_1] = [SSI_1] + OFFSET | SI_T[1] |
| [TSI_2] = [SSI_2] + OFFSET | SI_T[2] |
| [TSI_3] = [SSI_3] + OFFSET | SI_T[3] |
| … | … |
| [TSI_Y] = [SSI_Z] + OFFSET | SI_T[Z] |

112

FIG. 5D

| Array [X] | | |
|---|---|---|
| Scan Number: SN_M | Scan Line Number: SL_N | Transducer Spatial information: TSI_Z |
| [SN_1] | [SL_1]; …[SL_8]; … [SL_N] | [TSI_1] |
| [SN_2] | [SL_1]; …[SL_8]; … [SL_N] | [TSI_2] |
| [SN_3] | [SL_1]; …[SL_8]; … [SL_N] | [TSI_3] |
| … | … | … |
| [SN_M] | [SL_1]; …[SL_8]; … [SL_N] | [TSI_M] |

112

FIG. 5E

| Array [APEX] | | |
|---|---|---|
| Scan Number: SN_M | Scan Line Number: SL_1 | Transducer Spatial Information: TSI_Z |
| [SN_1] | [SL_1] | [TSI_1] |
| … | … | … |
| [SN_M] | [SL_1] | [TSI_M] |

| Array [MID] | | |
|---|---|---|
| Scan Number: SN_M | Scan Line Number: SL_N | Transducer Spatial Information: TSI_Z |
| [SN_1] | [SL_4] | [TSI_1] |
| … | … | … |
| [SN_M] | [SL_4] | [TSI_M] |

| Array [BASE] | | |
|---|---|---|
| Scan Number: SN_M | Scan Line Number: SL_N | Transducer Spatial Information: TSI_Z |
| [SN_1] | [SL_8] | [TSI_1] |
| … | … | … |
| [SN_M] | [SL_8] | [TSI_M] |

<u>112</u>

FIG. 5F

| Array [APEX-R] | | | |
|---|---|---|---|
| Scan Number: SN_M | Scan Line Number: SL_1 | Transducer Spatial Information: TSI_Z | Ray-Line Position: RL_P |
| [SN_1] | [SL_1] | [TSI_1] | +70.0 degrees |
| [SN_123] | [SL_1] | [TSI_123] | +69.5 degrees |
| [SN_246] | [SL_1] | [TSI_246] | +69.0 degrees |
| [SN_578] | [SL_1] | [TSI_578] | +68.5 degrees |
| [SN_832] | [SL_1] | [TSI_832] | +68.0 degrees |
| ... | ... | ... | ... |
| [SN_3,279] | [SL_1] | [TSI_1] | -70.0 degrees |

<u>112</u>

## FIG. 5G

| Array [B-mode] | | | |
|---|---|---|---|
| Scan Number: SN_M | Scan Line Number: SL_N | Transducer Spatial Information: TSI_Z | Ray-Line Position: R-L_P |
| [SN_1] | [SL_1]; ...[SL_8] | [TSI_1] | +0.0 degrees |
| [SN_123] | [SL_1]; ...[SL_8] | [TSI_123] | +0.0 degrees |
| [SN_246] | [SL_1]; ...[SL_8] | [TSI_246] | +0.0 degrees |
| [SN_578] | [SL_1]; ...[SL_8] | [TSI_578] | +0.0 degrees |
| [SN_832] | [SL_1]; ...[SL_8] | [TSI_832] | +0.0 degrees |
| ... | ... | ... | |
| [SN_3,279] | [SL_1]; ...[SL_8] | [TSI_3,279] | +0.0 degrees |

<u>112</u>

## FIG. 5H

FIG. 6

FIG. 7

FIG. 8

FIG. 9

134

238

$$x = (a + d) \cos(\theta)$$
$$y = (a + d) \sin(\theta)$$

232

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130066207 A1
- US 20130102903 A